# FASCICULE DE BREVET EUROPEEN

(11) **EP 3 762 433 B1**
(45) Date de publication et mention de la délivrance du brevet: **23.07.2025**
(21) Numéro de dépôt: 19715967.6
(22) Date de dépôt: 27.02.2019
(51) Int. Cl.: A61Q 19/00, A61K 8/81, C08L 33/26, A61K 8/06, C08F 220/58

(54) **LATEX INVERSE AUTO-INVERSIBLE EPAISSISSANT COMPRENANT COMME AGENT INVERSEUR DES ESPECES TENSIOACTIVES DE LA FAMILLE DES ESTERS DE POLYGLYCEROLS, ET LES COMPOSITIONS LE CONTENANT**
SELBSTINVERTIERENDER INVERTER LATEX, UMFASSEND ALS INVERTIERENDES MITTEL OBERFLÄCHENAKTIVE SPEZIES DER POLYGLYCERINESTERFAMILIE UND ZUSAMMENSETZUNGEN, DIE IHN UMFASSEN.
SELF-INVERTING REVERSE LATEX, COMPRISING AS INVERTING AGENT SURFACTANT SPECIES OF THE POLYGLYCEROL ESTER FAMILY AND COMPOSITIONS COMPRISING IT

(30) Priorité: 06.03.2018 FR 1851933
(43) Date de publication de la demande: 13.01.2021
(73) Titulaire: Société d'Exploitation de Produits pour les Industries Chimiques SEPPIC, 75321 Paris cedex 07 (FR)
(72) Inventeur: BODOC, Miruna, 81500 Lavaur (FR); GUILBOT, Jérôme, 81100 CASTRES (FR); DACOSTA, Georges, 81710 SAIX (FR); PIERRE, Aurélie, 78160 MARLY LE ROI (FR)
(74) Mandataire: Air Liquide
(86) Numéro de dépôt international: PCT/FR2019/050443
(87) Numéro de publication internationale: WO 2019/170979

(56) Documents cités:
- EP-A1- 1 055 451
- WO-A2-2009/156691
- US-A1- 2011 076 245
- US-A1- 2011 098 364
- US-B1- 6 375 959

## Description

L'invention concerne l'utilisation des latex inverses auto-inversibles comme épaississants utilisés pour préparer des formulations cosmétiques ou pharmaceutiques à usage topique, lesdits latex inverses auto-inversibles comprenant, comme agent inverseur, une composition tensioactive comprenant des esters de polyglycérols et du glycérol et/ou des oligomères du glycérol, ainsi que lesdites formulations ainsi préparées.

Les compositions cosmétiques et pharmaceutiques à usage topique, comprenant des phases polaires comme par exemple des phases aqueuses, alcooliques, hydroalcooliques ou hydro-glycoliques, nécessitent fréquemment l'utilisation de polymères modificateurs de rhéologie pour augmenter la viscosité desdites phases polaires, et plus généralement pour leur conférer un comportement rhéologique spécifique. Les modificateurs de rhéologie apportent à la fois une augmentation de la viscosité de la phase polaire, ainsi qu'une certaine consistance et/ou un effet stabilisant de la composition à usage topique à épaissir.

Parmi les agents modificateurs de rhéologie que l'on peut utiliser pour la préparation de ces compositions à usage topique, il y a des polymères synthétiques comme par exemple les polyélectrolytes anioniques, ou cationiques, ou ampholytes, linéaires ou ramifiés, réticulés ou non réticulés, se présentent sous deux formes physiques, la forme poudre et la forme liquide.

Lesdites compositions cosmétiques et pharmaceutiques à usage topique se présentent généralement sous la forme de gels aqueux, de gels hydroalcooliques, de gels hydro-glycoliques, d'émulsions ou de micro-émulsions ou de nano-émulsions de type eau-dans-huile ou de type huile-dans-eau ou de type eau-dans-huile-dans-eau ou de type huile-dans-eau-dans-huile.

Parmi les polyélectrolytes anioniques, ou cationiques, ou ampholytes, linéaires ou ramifiés, réticulés ou non réticulés se présentant sous une forme liquide, on distingue ceux connus sous le nom de « latex inverses auto-inversibles », qui sont des émulsions de type eau-dans-huile comprenant le polyélectrolyte, une phase aqueuse, une phase grasse composée au moins une huile, au moins un émulsionnant de type eau-dans-huile, au moins un émulsionnant de type huile-dans-eau. Dans les procédés de préparation de latex inverses auto-inversibles par la mise en œuvre d'une polymérisation radicalaire en émulsion inverse, les tensioactifs de type huile-dans-eau sont ajoutés à l'issue de l'étape de polymérisation. Leur ajout a pour objet de modifier et de régler la balance hydrophile-lipophile de l'émulsion eau-dans-huile comprenant le polymère (également nommée « latex inverse ») de façon à obtenir un mélange qui, une fois ajouté dans une phase polaire comme par exemple l'eau, changera de sens démulsion pour passer de la forme eau-dans-huile vers la forme huile-dans-eau, permettant alors de mettre en contact le polymère précédemment préparé avec la phase polaire à épaissir. Lors d'un tel phénomène physique, le polymère de type polyélectrolyte réticulé et/ou branché se déploie dans ladite phase polaire et forme un réseau tridimensionnel permettant à la phase polaire de gonfler, ce qui se manifeste par une augmentation de la viscosité de cette phase polaire. Le mélange comprenant le « latex inverse » et le tensioactif de type huile-dans-eau est nommé latex inverse auto-inversible et ledit tensioactif de type huile-dans-eau est nommé « inverseur » ou « agent inverseur ».

Les agents inverseurs couramment utilisés pour la préparation des latex inverses auto-inversibles sont des agents tensioactifs de type huile-dans-eau qui possèdent une valeur de HLB (« Hydrophilic Lipophilic Balance ») suffisamment élevée pour permettre de préparer des émulsions de type huile-dans-eau stables, généralement supérieur à 9 et inférieur à 16. Ils comprennent généralement une partie hydrophile constituée par un enchaînement de motifs d'oxyde d'éthylène et une partie consistant en une chaîne aliphatique hydrocarbonée de nature hydrophobe. Parmi ces agents inverseurs, il y a :
- Les alcools gras éthoxylés, dont la chaîne aliphatique hydrocarbonée comporte de 8 à 14 atomes de carbone et dont le nombre de motifs d'oxyde d'éthylène est compris entre 5 et 40, par exemple l'alcool laurique éthoxylé à 7 moles d'oxyde d'éthylène (nom INCI : Laureth-7), ou l'alcool tridécylique à 6 moles d'oxyde d'éthylène (nom INCI : trideceth-6) ;
- Les esters de sorbitan éthoxylés, dont la chaîne aliphatique hydrocarbonée comporte de 12 à 22 atomes de carbone et dont le nombre de motifs d'oxyde d'éthylène est compris entre 5 et 40, par exemple l'oléate de sorbitan éthoxylé à 20 moles d'oxyde d'éthylène commercialisé sous le nom commercial Montanox ^{™}80, ou le laurate de sorbitan éthoxylé à 20 moles d'oxyde d'éthylène commercialisé sous le nom commercial Montanox^{™}20 ;
- Les alkylphénols éthoxylés, par exemple les nonylphénols éthoxylés et les octylphénols éthoxylés ; ou
- Les huiles de ricin éthoxylées, par exemple l'huile de ricin éthoxylée à 40 moles d'oxyde d'éthylène commercialisée sous le nom de marque SIMULSOL ^{™}OL 50.

L'évolution des exigences des consommateurs et des dispositions réglementaires amènent les formulateurs de compositions cosmétiques à diminuer la proportion di'ngrédients comportant des motifs d'oxyde d'éthylène dans leurs formulations. Il existe donc un besoin de préparer des latex inverses auto-inversibles exempts de tensioactifs éthoxylés comme agents inverseurs.

Les demandes de brevet français publiées sous les numéros 2 794 034, 2 794 124, 2 808 447, 2 808 446 et 2 810 883 décrivent l'utilisation d'alkyl polyglycosides, dont la chaîne alkyle hydrocarbonée comporte d'un à trente atomes de carbone comme agents inverseurs pour préparer des latex inverses auto-inversibles, comme par exemple des mélanges d'alkyl polyglucosides dont les chaînes alkyles hydrocarbonées sont des chaînes décyle, dodécyle et tétradécyle comme le mélange commercialisé sous le nom de marque SIMULSOL^{™}SL 10, des chaînes dodécyle, tétradécyle et hexadécyle comme le mélange commercialisé sous le nom de marque SIMULSOL^{™}SL 26, des chaînes octyle et décyle comme le mélange commercialisé sous le nom de marque SIMULSOL^{™}SL 8, ou la chaîne undécylènyle comme le mélange commercialisé sous le nom de marque SIMULSOL^{™}SL 11W.

Cependant, la mise en oeuvre de tels composés pour préparer des latex inverses auto-inversibles, doit être réalisée à une température supérieure à leur point de fusion, généralement à 70°C, ce qui pose des problèmes d'augmentation de la viscosité du latex inverse et entraîne une certaine déstabilisation dudit latex inverse auto- inversible préparé. Dans certain cas, elle est réalisée en diluant préalablement lesdits alkyl polyglycosides dans l'eau pour disposer d'une forme liquide et manipulable à température ambiante. Ceci a parfois pour conséquence de diminuer la vitesse d'inversion desdits latex inverses auto-inversibles dans les phases polaires à épaissir, et donc de diminuer la productivité des procédés de préparation des formulations cosmétiques comprenant de tels agents épaississants.

La demande internationale publiée sous le numéro WO 2009/156691 divulgue l'utilisation d'esters de polyglycérol comme agents inverseurs pour préparer des latex inverses auto-inversibles, par exemple des esters de décaglycérol comme le monolaurate de décaglycérol, l'oléate de décaglycérol, le monocaprylate de décaglycérol ou le monomyristate de décaglycérol. Cependant, leur utilisation conduit à des latex inverses auto-inversibles pour lesquels la vitesse d'inversion dans les phases polaires à épaissir est trop lente et même diminue lorsque le latex inverse auto-inversible est stocké après sa préparation pendant plus d'un mois de préparation.

Les inventeurs ont donc cherché à mettre au point un nouveau système tensioactif inverseur de type huile-dans-eau, compatibles avec les normes environnementales en vigueur, en étant notamment exempts de motifs d'oxyde d'alkylène qui permettent de préparer des latex inverses auto-inversibles :
- qui puissent être utilisables facilement et notamment qui puissent être pompés à 25°C, qui aient une viscosité inférieure ou égale à 8.000 mPa.s, de préférence inférieure ou égale à 5.000 mPa.s, viscosité mesurée à 25°C à l'aide d'un viscosimètre Brookfield RVT et du mobile n°3 à la vitesse de 20 tours/minute,
- qui présentent un aspect lisse, exempts de grains ou de grumeaux, et
- qui possèdent de bonnes propriétés d'inversion dans des phases polaires c'est-à-dire induisant une vitesse d'inversion rapide et fiable.

L'invention a pour objet l'utilisation d'un latex inverse auto-inversible d'un polyélectrolyte anionique réticulé (P) comme agent épaississant et/ou émulsionnant et/ou stabilisant d'une composition topique cosmétique ou pharmaceutique, ledit latex inverse comprenant, pour 100% molaire :
**(a₁)** - d'une proportion supérieure ou égale à 30% molaire et inférieure ou égale à 100% molaire, d'unités monomériques issues de l'acide 2-méthyl 2-[(1-oxo 2-propènyl) amino] 1-propanesulfonique sous forme acide libre ou partiellement ou totalement salifiée ;
**(a₂)** - optionnellement d'une proportion supérieure à 0% molaire et inférieure ou égale à 70% molaire, d'unités monomériques issues d'au moins un monomère choisi parmi les éléments du groupe constitué par l'acide acrylique, l'acide méthacrylique, l'acide 2-carboxyéthyl acrylique, l'acide itaconique, l'acide maléique, l'acide 3-méthyl 3-[(1-oxo 2-propènyl) amino] butanoïque, la fonction carboxylique desdits monomères étant sous forme acide libre, partiellement salifiée ou totalement salifiée et/ou parmi les éléments du groupe constitué par l'acrylate de (2-hydroxy éthyle), l'acrylate de (2,3-dihydroxy propyle), le méthacrylate de (2-hydroxy éthyle), le méthacrylate de (2,3-dihydroxypropyle), ou le vinyl pyrrolidone ;
**(a₃)** - d'une proportion supérieure à 0% molaire et inférieure ou égale à 1% molaire d'unités monomériques issues d'au moins un monomère de réticulation (AR) diéthylénique ou polyéthylénique;
la somme des dites proportions molaires en unités monomériques selon a₁), a₂) et a₃) étant égale à 100% molaire;
ledit latex inverse auto-inversible étant une émulsion de type eau-dans-huile (E) comprenant pour 100% de sa masse :
**a)** - de 10% massique à 90% massique, dudit polyélectrolyte anionique réticulé (P) ;
**b) -** De 5% massique à 50% massique, d'une phase grasse constituée d'au moins une huile (H),
**c)** - De 1% massique à 50% massique d'eau,
**d) -** De 0,5% massique à 10% massique d'un système émulsionnant de type eau-dans-huile (S₁), et
**e)** - De 2% massique à 10% massique d'un système émulsionnant de type huile-dans-eau (S₂) ;
   la somme des proportions massiques en composés selon a), b), c), d) et e) étant égale à 100% massique ;
   ledit latex inverse auto-inversible étant **caractérisée en ce que** ledit système émulsionnant de type huile-dans-eau (S₂)comprend pour 100% de sa masse :
**f)** - Une proportion supérieure ou égale à 50% massique et inférieure ou égal à 100% d'une composition (Ce) qui comprend pour 100% de sa masse :
   **e₁)** - De 10% massique à 60% massique, plus particulièrement de 15% massique à 60% massique et tout particulièrement de 15% massique à 50% d'au moins un composé de formule (I) :

      HO-[CH₂-CH(OH)-CH₂-O]ₙ-H (I)

      dans laquelle n représente un nombre entier supérieur ou égal à un et inférieur ou égal à quinze ;
   **e₂)** - De 40% massique à 90 % massique, plus particulièrement de 40% massique à 85 % massique et tout particulièrement de 50% massique à 85% massique d'au moins un composé de formule (II) :

      R₁-(C=O)-[O-CH₂-CH(OH)-CH₂]ₚ-OH (II),

      dans laquelle p, différent ou identique à n, représente un nombre entier supérieur ou égal à un et inférieur ou égal à quinze ; et dans laquelle le groupe R₁-(C=O)- représente un radical aliphatique saturé ou insaturé, linéaire ou ramifié, comportant de six à vingt-deux atomes de carbone, et optionnellement
   **e₃)** - Jusquà 30% massique, plus particulièrement de 0% massique à 25% massique et tout particulièrement de 0% massique à 20% massique d'au moins une composition (C₁₁) représentée par la formule (III) :

      HO-[CH₂-CHOH-CH₂-O-]_{q}-(G)ᵣ-H (III),

      dans laquelle q différent ou identique à n, représente un nombre entier supérieur ou égal à un et inférieur ou égal à 3, G représente le reste d'un sucre réducteur et r représente un nombre décimal supérieur ou égal à 1,05 et inférieur ou égal à 5,00,
      ladite composition (C₁₁) consistant en un mélange des composés de formules (III₁), (III₂), (III₃), (III₄) et (III₅) :

         HO-[CH₂-CHOH-CH₂-O-]_{q}-O-(G)₁-H (III₁),

         HO-[CH₂-CHOH-CH₂-O-]_{q}-O-(G)₂-H (III₂),

         HO-[CH₂-CHOH-CH₂-O-]_{q}-O-(G)₃-H (III₃),

         HO-[CH₂-CHOH-CH₂-O-]_{q}-O-(G)₄-H (III₄),

         HO-[CH₂-CHOH-CH₂-O-]_{q}-O-(G)₅-H (III₅),

         en des proportions molaires en dits composés de formules (III₁), (III₂), (III₃), (III₄) et (III₅) respectivement égales à a₁, a₂, a₃, a₄ et a₅, telles que la somme (a₁+ a₂ + a₃ + a₄ + a₅) est égale à un, et que la somme (a₁ + 2a₂ + 3a₃ + 4a₄ + 5a₅) est égale à r ;
      la somme des proportions massiques en composés selon e₁), e₂) et e₃) étant égale à 100% massique.

Au sens de la présente invention, par polyélectrolyte anionique réticulé (P), on désigne pour le polymère (P), un polyélectrolyte non linéaire qui se présente à létat d'un réseau tridimensionnel insoluble dans l'eau, mais gonflable à l'eau et conduisant alors à l'obtention d'un gel chimique.

Au sens de la présente invention, le terme «salifié» indique que la fonction acide présente dans un monomère se trouve sous une forme anionique associée sous forme de sel à un cation, notamment les sels de métaux alcalins, tels que les cations du sodium ou du potassium, ou comme les cations de base azotés tels que le sel d'ammonium, le sel de lysine ou le sel de monoéthanolamine (HOCH₂-CH₂-NH₄⁺). Il s'agit de préférence des sels de sodium ou d'ammonium.

Selon un aspect particulier de la présente invention, ledit latex inverse auto-inversible tel que défini ci-dessus comprend de 20% massique à 90% massique, et plus particulièrement de 30% massique à 90% massique, plus particulièrement de 30% massique à 80% massique, et encore plus particulièrement de 33% massique à 80% massique dudit polyélectrolyte anionique (P) réticulé.

Selon un autre aspect particulier de la présente invention, la proportion molaire en unités monomériques issues de acide 2-méthyl 2-[(1-oxo 2-propènyl) amino] 1-propanesulfonique sous forme acide libre ou partiellement ou totalement salifiée présentes dans ledit polyélectrolyte anionique réticulé (P) est supérieure ou égale à 32% molaire et inférieure ou égale à 100% molaire, plus particulièrement supérieure ou égale à 40% molaire et inférieure ou égale à 100% molaire.

Selon un aspect particulier de la présente invention, l'acide 2-méthyl 2-[(1-oxo 2-propènyl) amino] 1-propanesulfonique est sous forme de sel de de sodium ou d'ammonium.

Selon un autre aspect particulier de la présente invention, ledit polyélectrolyte anionique réticulé (P) est issu de la polymérisation, pour 100% molaire :
**(a₁)** - d'une proportion supérieure ou égale à 32% molaire et inférieure à 100% molaire, plus particulièrement supérieure ou égale à 40% molaire et inférieure ou égale à 100% molaire, d'unités monomériques issues d'un monomère possédant une fonction acide fort, partiellement salifiée ou totalement salifiée, plus particulièrement d'un sel de sodium ou un sel d'ammonium de l'acide 2-méthyl 2-[(1-oxo 2-propènyl) amino] 1-propanesulfonique ; et
**(a₂)** - d'une proportion supérieure à 0% molaire et inférieure ou égale à 68% molaire, plus particulièrement supérieur à 0% molaire et inférieure ou égale à 60% molaire, d'unités monomériques issues d'au moins un monomère choisi parmi les éléments du groupe constitué par l'acide acrylique, l'acide méthacrylique, l'acide 2-carboxyéthyl acrylique, l'acide itaconique, l'acide maléique, l'acide 3-méthyl 3-[(1-oxo 2-propènyl) amino] butanoïque, la fonction carboxylique desdits monomères étant sous forme acide, partiellement salifiés ou totalement salifiés, et/ou parmi les éléments du groupe constitué par l'acrylate de (2-hydroxy éthyle), l'acrylate de (2,3-dihydroxy propyle), le méthacrylate de (2-hydroxy éthyle), le méthacrylate de (2,3-dihydroxypropyle), et le vinyl pyrrolidone ; et
**(a₃)** - d'une proportion supérieure à 0% molaire et inférieure ou égale à 1% molaire d'unités monomériques issues d'au moins un monomère de réticulation (AR) diéthylénique ou polyéthylénique ; étant entendu que la somme des proportions molaires des unités monomériques (a₁), (a₂) et (a₃) est égale à 100%.

Par au moins un monomère de réticulation (AR) diéthylènique ou polyéthylénique, on désigne, dans la définition dudit polyélectrolyte anionique réticulé (P), on désigne notament un monomère choisi le méthylène-bis(acrylamide), le diméthacrylate d'éthylèneglycol le diacrylate de diéthylèneglycol, le diacrylate d'éthylèneglycol, le diallyl urée, le triallylamine, le triméthylol propanetriacrylate ou le méthylène-bis(acrylamide) ou un mélange de ces composés, l'acide diallyoxyacétique ou un de ses sels comme le diallyloxyacétate de sodium, ou un mélange de ces composés ; et plus particulièrement un monomère choisi parmi le diméthacrylate d'éthylèneglycol, le triallylamine, le triméthylol propanetriacrylate ou le méthylène-bis(acrylamide) ou un mélange ces composés.

Selon un autre aspect particulier, ledit monomère de réticulation (AR) est mis en oeuvre dans une proportion molaire inférieure ou égale à 0,5%, plus particulièrement inférieure ou égale à 0,25% et tout particulièrement inférieure ou égales à 0,1 % ; elle est plus particulièrement supérieure ou égales à 0,005% molaire.

Selon un autre aspect particulier de la présente invention, ledit polyélectrolyte anionique réticulé (P) est un homopolymère de l'acide 2-méthyl 2-[(1-oxo 2-propènyl) amino] 1-propanesulfonique partiellement ou totalement salifié sous forme de sel de sodium ou de sel d'ammonium, réticulé par le triallylamine et/ou le méthylène-bis(acrylamide), un copolymère de l'acide 2-méthyl 2-[(1-oxo 2-propènyl) amino] 1-propanesulfonique et de l'acide acrylique partiellement ou totalement salifiés sous forme de sel de sodium ou de sel d'ammonium, réticulé par le triallylamine et/ou le méthylène-bis(acrylamide) ; un copolymère de l'acide 2-méthyl 2-[(1-oxo 2-propènyl) amino] 1-propanesulfonique (γ) partiellement ou totalement salifié sous forme de sel de sodium, et d'acide acrylique (δ) partiellement ou totalement salifié sous forme de sel de sodium dans un rapport molaire (γ)/(δ) supérieur ou égal à 30/70 et inférieur ou égal à 90/10 ; réticulé par le triallylamine et/ou le méthylène-bis(acrylamide) ; ou un copolymère de l'acide 2-méthyl 2-[(1-oxo 2-propènyl) amino] 1-propanesulfonique (γ) partiellement ou totalement salifié sous forme de sel de sodium, et de l'acide acrylique (δ)partiellement ou totalement salifié sous forme de sel de sodium dans un rapport molaire (γ)/(δ)supérieur ou égal à 40/60 et inférieur ou égal à 90/10, réticulé par le triallylamine et/ou le méthylène-bis(acrylamide).

Par huile (H), on désigne dans la définition dudit latex inverse auto-inversible, notamment :
- Les alcanes linéaires comportant de onze à dix-neuf atomes de carbone ;
- Les alcanes ramifiés, comportant de sept à quarante atomes de carbone, comme l'isodécane, l'isopentadécane, l'isohexadécane, l'isoheptadécane, l'isooctadécane, l'isononadécane ou l'isoeicosane, ou des mélanges de certains d'entre eux comme ceux cités ci-après et identifiés par leur nom INCI : C₇₋₈ isoparaffin, C₈₋₉ isoparaffin, C₉₋₁₁ isoparaffin, C₉₋₁₂ isoparaffin, C₉₋₁₃ isoparaffin, C₉₋₁₄ isoparaffin, C₉₋₁₆ isoparaffin, C₁₀₋₁₁ isoparaffin, C₁₀₋₁₂ isoparaffin, C₁₀₋₁₃ isoparaffin, C₁₁₋₁₂ isoparaffin, C₁₁₋₁₃ isoparaffin, C₁₁₋₁₄ isoparaffin, C₁₂₋₁₄ isoparaffin, C₁₂₋₂₀ isoparaffin, C₁₃₋₁₄ isoparaffin, C₁₃₋₁₆ isoparaffin ;
- Les cyclo-alcanes optionnellement substitués par un ou plusieurs radicaux alkyles linéaires ou ramifies ;
- Les huiles blanches minérales, comme celles commercialisées sous les noms suivants : Marcol^{™}52, Marcol^{™}82, Drakeol^{™}6VR, Eolane^{™}130, Eolane^{™} 150 ;
- Lhémisqualane (ou 2,6,10-trimethyl- dodécane ; numéro CAS : 3891-98-3), le squalane (ou 2,6,10,15,19,23-hexamethyltetracosane), le polyisobutène hydrogéné ou le polydécène hydrogéné ;
- Les mélanges d'alcanes comportant de 15 à 19 atomes de carbone, lesdits alcanes étant des alcanes linéaires, des alcanes ramifiés et des cyclo-alcanes, et plus particulièrement le mélange (M₁) qui comprend pour 100% de sa masse, une proportion massique en alcanes ramifiés supérieure ou égale à 90% et inférieure ou égale à 100% ; une proportion massique en alcanes linéaires supérieure ou égale à 0% et inférieure ou égale à 9%, et plus particulièrement inférieure à 5% et une proportion massique en cyclo-alcanes supérieure ou égale à 0% et inférieure ou égale à 1%, par exemple les mélanges commercialisés sous les noms Emogreen^{™}L15 ou Emogreen^{™}L19 ;
- Les éthers d'alcool gras de formule (IV) :

   Z₁-O-Z₂ (IV),

   dans laquelle Z₁ et Z₂ identiques ou différents, représentent un radical alkyle linéaire ou ramifié comportant de cinq à dix-huit atomes de carbone, par exemple les dioctyl éther, didécyl éther, didodécyl éther, dodécyl octyl éther, dihexadécyl éther, (1,3-diméthyl butyl) tétradécyl éther, (1,3-diméthyl butyl) hexadécyl éther, le bis(1,3-diméthyl butyl) éther ou le dihexyl éther.
- Les mono-esters d'acides gras et d'alcools de formule (V) :

   R'₁-(C=O)-O-R'₂ (V),

   dans laquelle R'₁-(C=O) représente un radical acyle, saturé ou insaturé, linéaire ou ramifié, comportant de huit à vingt-quatre atomes de carbone, et R2 représente, indépendamment de R'₁, une chaîne hydrocarbonée saturée ou insaturée, linéaire ou ramifiée comportant de un à vingt-quatre atomes de carbone, par exemple les laurate de méthyle, laurate d'éthyle, laurate de propyle, laurate d'isopropyle, laurate de butyle, laurate de 2-butyle, laurate d'hexyle, cocoate de méthyle, cocoate d'éthyle cocoate de propyle, cocoate d'isopropyle, cocoate de butyle, cocoate de 2-butyle, cocoate d'hexyle, myristate de méthyle, myristate d'éthyle, myristate de propyle, le myristate d'isopropyle, le myristate de butyle, le myristate de 2-butyle, le myristate d'hexyle, le myristate d'octyle, le palmitate de méthyle, le palmitate d'éthyle, le palmitate de propyle, le palmitate d'isopropyle, le palmitate de butyle, le palmitate de 2-butyle, le palmitate d'éthyle, le palmitate d'octyle, l'oléate de méthyle, l'oléate d'éthyle, l'oléate de propyle, l'oléate d'isopropyle, l'oléate de butyle, l'oléate de 2-butyle, l'oléate d'hexyle, l'oléate d'octyle, le stéarate de méthyle, le stéarate d'éthyle, le stéarate de propyle, le stéarate d'isopropyle, le stéarate de butyle, le stéarate de 2-butyle, le stéarate d'hexyle, le stéarate d'octyle, l'isostéarate de méthyle, l'isostéarate d'éthyle, l'isostéarate de propyle, l'isostéarate d'isopropyle, l'isostéarate de butyle, l'isostéarate de 2-butyle, l'isostéarate d'hexyle, l'isostéarate d'isostéaryle ;
- Les di-esters d'acides gras et de glycérol de formule (VI) et de formule (VII) :

   R'₃-(C=O)-O-CH₂-CH(OH)-CH₂-O-(C=O)-R'₄ (VI)

   R'₅-(C=O)-O-CH₂-CH[O-(C=O)-R'₆]-CH₂-OH (VII),

   formules (VI) (VII) dans lesquelles R'₃-(C=O), R'₄-(C=O), R'₅-(C=O), R'₆-(C=O), identiques ou différents, représentent un groupement acyle, saturé ou insaturé, linéaire ou ramifié, comportant de huit à vingt-quatre atomes de carbone ;
- Les tri-esters d'acides gras et de glycérol de formule (VIII) :

   R'₇-(C=O)-O-CH₂-CH[O-(C=O)-R"₈]-CH₂-O-(C=O)-R"₉ (VIII),

   dans laquelle R'₇-(C=O), R's-(C=O) et R'₉-(C=O), identiques ou différents, représentent un groupement acyle, saturé ou insaturé, linéaire ou ramifié, comportant de huit à vingt-quatre atomes de carbone.

Selon un autre aspect particulier de la présente invention, ladite huile (H) est choisie parmi l'undécane, le tridécane, l'isodécane ou l'isohexadécane, les mélanges d'alcanes et d'isoalcanes et de cycloalcanes comme le mélange (M ₁) tel que défini précédemment et les mélanges commercialisés sous les noms Emogreen^{™}L15, Emogreen^{™}L19, Emosmart^{™}L15, Emosmart^{™}L19, Emosmart^{™}V21, Isopar^{™}L ou Isopar^{™}M ; les huiles blanches minérales commercialisées sous les noms Marcol^{™}52, Marcol^{™}82, Drakeol^{™}6VR, Eolane^{™}130 ou Eolane^{™}150 ; l'hémisqualane, le squalane, le polyisobutène hydrogéné ou le polydécène hydrogéné ; le dioctyl éther ou le didécyl éther ; le myristate d'isopropyle, le palmitate d'hexyle, le palmitate d'octyle, l'isostéarate d'isostéaryle, l'octanoyl/décanoyl triglycéride, l'hexadécanoyl/octadécanoyl triglycéride, les triglycérides issus de l'huile de colza, de l'huile de tournesol, de l'huile de lin ou de l'huile de palme.

Dans ledit latex inverse auto-inversible, le système émulsionnant (S₁) de type eau-dans-huile est constitué soit d'un seul tensioactif émulsionnant soit d'un mélange de tensioactifs émulsionnants, à condition que ledit système émulsionnant (S₁) résultant ait une valeur de HLB suffisamment faible pour induire la formation d'émulsions de type eau-dans-huile.

Comme tensioactif émulsionnant de type eau-dans-huile, il y a par exemple les esters d'anhydro hexitol et d'acides carboxyliques aliphatiques, linéaires ou ramifiés, saturés ou insaturés, comportant de 12 à 22 atomes de carbone éventuellement substitués avec un ou plusieurs groupes hydroxyles, et plus particulièrement les esters d'anhydro hexitol choisis parmi les anhydro-sorbitols et les anhydro-mannitols et d'acides carboxyliques aliphatiques, linéaires ou ramifiés, saturés ou insaturés, comportant de 12 à 22 atomes de carbone éventuellement substitués avec un ou plusieurs groupes hydroxyles.

Selon un autre aspect particulier de la présente invention, ledit système émulsionnant (S₁) de type eau-dans-huile est choisi parmi les éléments du groupe constitué par le laurate de sorbitan, par exemple celui commercialisé sous le nom Montane^{™}20, le palmitate de sorbitan, par exemple celui commercialisé sous le nom Montane^{™}40, le stéarate de sorbitan, par exemple celui commercialisé sous le nom Montane^{™}60, l'oléate de sorbitan, par exemple celui commercialisé sous le nom Montane^{™}80, le sesquioléate de sorbitan, par exemple celui commercialisé sous le nom Montane^{™}85, le trioléate de sorbitan, par exemple celui commercialisé sous le nom Montane^{™}83, l'isolaurate de sorbitan, l'isostéarate de sorbitan, par exemple celui commercialisé sous le nom Montane^{™}70, le laurate de mannitan, l'oléate de mannitan, ou un mélange de ces esters ; les polyesters de poids moléculaire compris entre 1000 et 3000 et issus de la condensation entre un acide poly(isobutényl) succinique ou son anhydride, tels que l'HYPERMER^{™} 2296, ou le mélange commercialisé sous le nom de marque SIMALINE^{™}IE 501 A, les polyhydroxystéarates de polyglycols de formule (IX) : 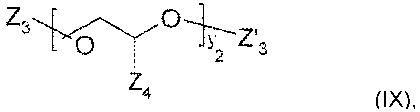 formule (IX) dans laquelle y₂ représente un nombre entier supérieur ou égal à 2 et inférieur ou égal à 50, Z₄ représente l'atome d'hydrogène, le radical méthyle, ou le radical éthyle, Z₃ représente un radical de formule (X) : 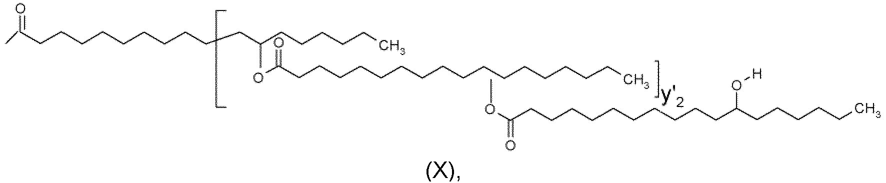 formule (X) dans laquelle y'₂ représente un nombre entier supérieur ou égal à 0 et inférieur ou égal à 10, plus particulièrement supérieur ou égal à 1 et inférieur ou égal à 10 et Z'₃ représente un radical de formule (X) telle que définie ci-dessus, avec Z₃' identique ou différent de Z₃, ou l'atome d'hydrogène

Comme exemple de tensioactif émulsionnant de type eau-dans-huile de formule (IX) que l'on peut utiliser pour préparer le système émulsionnant (S ₁), il y a le PEG-30 dipolyhydroxystéarate commercialisé sous le nom SIMALINE^{™} WO, ou bien les mélanges comprenant le PEG-30 dipolyhydroxystéarate et commercialisés sous les noms SIMALINE^{™}IE 201 A et SIMALINE^{™}IE 201 B, ou encore le mélange comprenant du Triméthylolpropane-30 tripolyhydroxystéarate commercialisé sous le nom SIMALINE^{™}IE 301 B.

Dans ledit latex inverse auto-inversible, le système émulsionnant (S₂) de type huile-dans-eau est constitué soit de la seule composition (Cₑ), soit d'un mélange de ladite composition (Cₑ) avec un ou plusieurs autres tensioactifs émulsionnants, à condition que ledit système émulsionnant (S₂) résultant ait une valeur de HLB suffisamment élevée pour induire la formation d'émulsions de type huile-dans-eau.

Selon un autre aspect plus particulier de la présente invention, ledit latex inverse auto-inversible tel que défini précédemment est caractérisé en ce que dans la formule (I), n représente un nombre entier supérieur ou égal à un et inférieur ou égal à dix, et en **ce que** dans la formule (II), p, identique ou différent de n, représente un nombre entier supérieur ou égal à un et inférieur ou égal à dix, et le groupe R₄-(C=O)- est choisi parmi les radicaux octanoyle, décanoyle, w-undécylènoyle, dodécanoyle, tétradécanoyle, hexadécanoyle, octadécanoyle, 9-octadécènoyle ou 9,12-octadécadiènoyle.
Selon un autre aspect encore plus particulier de la présente invention, dans les dites formules (I) et (II) telles que définies précédemment, n est égal à 10, p est égal à 10, et le groupe R₁-(C=O)- est le radical dodécanoyle ; n est égal à 6, p est égal à 10, et le groupe R₁-(C=O)- est le radical dodécanoyle ; n est égal à 6, p est égal à 6, et le groupe R₁-(C=O)- est le radical dodécanoyle ou n est égal à 1, p est égal à 10, et le groupe R₁-(C=O)- est le radical dodécanoyle.

Selon un autre aspect plus particulier de la présente invention, ledit latex inverse auto-inversible tel que défini précédemment est **caractérisé en ce que** dans ledit système émulsionnant de type huile-dans-eau (S₂) ladite composition (Cₑ) telle que définie précédemment, consiste en, pour 100% de sa masse :
**e₁)** - De 10% massique à 60% massique d'au moins un composé de formule (I) telle que définie précédemment et
**e₂)** - De 40% massique à 90 % massique d'au moins un composé de formule (II) telle que définie précédemment.

Par sucre réducteur, on désigne dans la formule (III) telle que définie précédemment, les dérivés saccharidiques qui ne présentent pas dans leurs structures de liaison glycosidique établie entre un carbone anomérique et l'oxygène d'un groupement acétal tels qu'ils sont définis dans l'ouvrage de référence : "Biochemistry", Daniel Voet/Judith G. Voet, p. 250, John Wyley & Sons, 1990. La structure oligomérique (G)ₓ peut se présenter sous toutes formes d'isoméries, qu'il s'agisse d'isomérie optique, d'isomérie géométrique ou d'isomérie de position ; elle peut aussi représenter un mélange d'isomères.

Selon un autre aspect plus particulier de la présente invention, dans la composition (Cₑ) telle que définie précédemment, G représente, dans la formule (III) telle que définie précédemment, le reste d'un sucre réducteur choisi parmi les restes du glucose, du dextrose, du saccharose, du fructose, de l'idose, du gulose, du galactose, du maltose, de l'isomaltose, du maltotriose, du lactose, du cellobiose, du mannose, du ribose, du xylose, de l'arabinose du lyxose, de l'allose, de l'altrose du dextrane ou du tallose. Ledit reste G représente encore plus particulièrement dans la formule (III) telle que définie précédemment, un sucre réducteur choisi parmi les restes du glucose, du xylose et de l'arabinose.

Par la formule (III) :

HO-CH₂-(CHOH)_{q}-CH₂-O-(G)ᵣ-H,

représentant la composition (C₁₁), on signifie que cette composition (C₁₁) consiste essentiellement en un mélange de composés représentés par les formules (III₁), (III₂), (III₃), (III₄) et (III₅) :

HO-CH₂-(CHOH)_{q}-CH₂-O-(G)₁-H (III₁),

HO-CH₂-(CHOH)_{q}-CH₂-O-(G)₂-H (III₂),

HO-CH₂-(CHOH)_{q}-CH₂-O-(G)₃-H (III₃),

HO-CH₂-(CHOH)_{q}-CH₂-O-(G)₄-H (III₄),

HO-CH₂-(CHOH)_{q}-CH₂-O-(G)₅-H (III₅),

dans les proportions molaires respectives a₁, a₂, a₃, a₄ et a₅, telles que la somme a₁ + a₂ + a₃ + a₄ + a₅ est égale à 1 et que la somme a₁ + 2a₂ + 3a₃ + 4a₄ + 5a₅ est égale à r.

Par essentiellement, on indique dans la définition qui précède, que la présence d'un ou de plusieurs composés de formule (III_{w}) avec w supérieur à 5 n'est pas exclue au sein de la composition (C₁₁), mais que si présence il y a, elle l'est en des proportions minimes qui n'entraînent aucune modification substantielle des propriétés de ladite composition (C₁₁). Dans la formule (III) telle que définie ci-dessus, le groupe HO-CH₂-(CHOH)_{q}-CH₂-O- est lié à (G)ᵣ par le carbone anomérique du reste saccharide, de manière à former une fonction acétal.

Selon un aspect plus particulier de la présente invention, dans la formule (III) représentant la composition (C₁₁) telle que définie précédemment, r représente un nombre décimal supérieur ou égal à 1,05 et inférieur ou égal à 3, plus particulièrement supérieur ou égal à 1,15 et inférieur ou égal à 2,5.

Selon un aspect plus particulier de la présente invention, ledit latex inverse auto-inversible tel que défini précédemment est **caractérisé en ce que** dans la formule (III) telle que définie précédemment, q est égal à un, G représente le reste du glucose et r représente un nombre décimal supérieur ou égal à 1,05 et inférieur ou égal à 2,5.

Selon un autre aspect particulier de la présente invention, ledit latex inverse auto-inversible tel que défini précédemment est **caractérisé en ce que** dans ledit système émulsionnant de type huile-dans-eau (S₂), ladite composition (Cₑ) telle que définie précédemment consiste en, pour 100% de sa masse :
**e₁)** - De 5% massique à 15% massique d'au moins un composé de formule (I) telle que définie précédemment,
**e₂)** - De 60% massique à 80 % massique d'au moins un composé de formule (II) telle que définie précédemment, et
**e₃)** - De 5% à 15% massique d'au moins une composition (C ₁₁) représentée par la formule (III) telle que définie précédemment.

Selon un autre aspect particulier de la présente invention, ledit système émulsionnant (S₂) de type huile-dans-eau comprend pour 100% de sa masse, au moins 75% en masse de ladite composition (Cₑ) telle que définie précédemment.

Selon un aspect tout particulier ledit latex inverse tel que défini précédemment est **caractérisé en ce que** ledit système émulsionnant de type huile-dans-eau (S₂) est ladite composition (Cₑ) telle que définie précédemment.

Ledit latex inverse auto-inversible est préparé par la mise en oeuvre d'un procédé dit de « polymérisation en émulsion inverse », bien connu de l'homme du métier, et qui comprend les étapes suivantes :
- Une étape a) de préparation d'une phase aqueuse comprenant de l'eau, les monomères hydrosolubles et optionnellement le monomère de réticulation (AR), ainsi que des additifs couramment utilisés comme par exemple des agents séquestrants comme l'éthylènediaminetétracétique (EDTA) sous sa forme sodique, ou le sel de penta-sodium de l'acide penta acétique du diéthylènetramine (commercialisé sous le nom de marque Versenex^{™}80) ;
- Une étape b) de mélange de la phase huileuse (H) avec le système émulsionnant de type eau-dans-huile (S₁) ;
- Une étape c) de mélange de la phase aqueuse et de la phase huileuse, préparées lors des précédentes étapes, et d'émulsification à l'aide d'un mobile de type rotor-stator ;
- Une étape d) d'inertage à l'azote ;
- Une étape e) d'amorçage de la réaction de polymérisation par introduction dans l'émulsion formée en c), d'un initiateur de radicaux libres et d'éventuellement un co-initiateur ; puis on la laisse se dérouler,
- Une étape f) d'introduction du système émulsionnant (S ₂) de type huile-dans-eau tel que défini précédemment à une température inférieure ou égale à 50°C.

Selon un aspect particulier du procédé tel que défini précédemment, la réaction de polymérisation de l'étape e) est amorcée par un couple oxydo-réducteur générateur d'ions hydrogénosulfite (HSO₃⁻), tel que le couple hydroperoxyde de cumène - métabisulfite de sodium (Na₂S₂O₅) ou le couple hydroperoxyde de cumène-chlorure de thionyle (SOCl₂) à une température inférieure ou égale à 10°C, si désiré accompagné d'un agent co-initiateur de polymérisation tel que par exemple l'azo-bis(isobutyronitrile) puis conduite soit de manière quasi adiabatique jusqu'à une température supérieure ou égale à 50° C, soit en contrôlant la température.

Selon un autre aspect particulier du procédé tel que défini précédemment, le milieu réactionnel issu de l'étape e), est concentré par distillation, avant la mise en œuvre de l'étape f).

Selon un autre aspect particulier du procédé tel que défini précédemment, le milieu réactionnel issu de l'étape e) ou de l'étape f) subit une étape de séchage par atomisation dans une installation adaptée.

Selon un autre aspect particulier du procédé tel que défini précédemment, la phase aqueuse préparée à l'étape
a) peut comprendre des agents réducteurs de chaîne, destinés à réduire la longueur des chaînes polymériques formées et à augmenter le taux de branchement sur le polymère, de façon à modifier les propriétés rhéologiques.

Parmi les agents réducteurs de chaîne adaptés au procédé tel que défini précédemment, il y a le méthanol, li'sopropanol, le butylène glycol, le 2-mercapto éthanol, l'acide thioglycolique, l'acide formique ou ses sels.

Selon un autre aspect particulier, ladite utilisation consiste à stabiliser une émulsion de type huile-dans-eau, ou de type eau-dans-huile, en conférant un aspect homogène à ladite émulsion pendant le stockage dans différentes conditions, et plus particulièrement à 25°C pendant une durée au moins égale à un mois, et plus particulièrement à 4°C pendant une durée au moins égale à un mois, et plus particulièrement à 45°C pendant une durée au moins égale à un mois.

Selon un autre aspect particulier, ladite utilisation consiste à stabiliser des particules solides dans des compositions topique cosmétiques, dermopharmaceutiques ou pharmaceutiques. Ces particules solides à suspendre peuvent revêtir différentes géométries, régulières ou irrégulières, et se présenter sous forme de perles, de billes, de tiges, de paillettes, de lamelles ou de polyèdres. Ces particules solides se caractérisent par un diamètre moyen apparent compris entre un micromètre et cinq millimètres, plus particulièrement entre dix micromètres et un millimètre.

Parmi les particules solides qui peuvent être mises en suspension et stabilisées par le latex inverse auto-inversible telle que définie précédemment dans des compositions topiques cosmétiques, dermopharmaceutiques ou pharmaceutiques, il y a les micas, l'oxyde de fer, l'oxyde de titane, l'oxyde de zinc, l'oxyde d'aluminium, le talc, la silice, le kaolin, les argiles, le nitrure de bore, le carbonate de calcium, le carbonate de magnésium, l'hydrogénocarbonate de magnésium, les pigments colorés inorganiques, les polyamides comme le nylon-6, les polyéthylènes, les polypropylènes, les polystyrènes, les polyesters, les polymères acryliques ou méthacryliques comme les polyméthylméthacrylates, le polytétrafluoroéthylène, les cires cristallines ou microcristallines, des sphères poreuses, le sulphide de sélénium, le pyrithione de zinc, les amidons, les alginates, les fibres de végétaux, les particules de Loofah, les particules d'éponges

L'invention a également pour objet une composition cosmétique topique (F) ou une composition pharmaceutique topique (G), **caractérisée en ce qu'**elle comprend comme agent épaississant, pour 100% de sa masse totale entre 0,1% et 10% massique dudit latex inverse auto-inversible tel que défini précédemment.

L'expression "topique" utilisée dans les définitions des dites compositions (F) et (G), signifie qu'elles sont mises en oeuvre par application sur la peau, les cheveux, le cuir chevelu ou les muqueuses, qu'il s'agisse d'une application directe dans le cas d'une préparation cosmétique, dermocosmétique, dermopharmaceutique ou pharmaceutique ou d'une application indirecte par exemple dans le cas d'un produit de soin corporel sous forme de lingette en textile ou en papier ou de produits sanitaires destinés à être en contact avec la peau ou les muqueuses.

Lesdites compositions (F) et (G), se présentent généralement sous forme d'une solution aqueuse ou hydro-alcoolique ou hydro-glycolique, sous forme d'une suspension, d'une émulsion, d'une microémulsion ou d'une nano-émulsion, qu'elles soient de type eau-dans- huile, huile-dans-eau, eau-dans-huile-dans-eau ou huile-dans-eau-dans-huile.

Lesdites compositions (F) et (G), peut être conditionnée dans un flacon, dans un dispositif de type "flacon" pompe, sous forme pressurisées dans un dispositif aérosol, dans un dispositif muni d'une paroi ajourée comme une grille ou dans un dispositif muni d'un applicateur à billes (dit "roll-on").

De façon générale, lesdites compositions (F) et (G) comportent également des excipients et ou des principes actifs habituellement mis en oeuvre dans le domaine des formulations à usage topique, en particulier cosmétiques, dermocosmétiques, pharmaceutiques ou dermopharmaceutiques, comme les tensioactifs épaississants et/ou gélifiants, les stabilisants, les composés filmogènes, les agents hydrotropes, les agents plastifiants, les agents émulsionnants et co-émulsionnants, les agents opacificants, les agents nacrants, les agents surgraissants, les séquestrants, les agents chélatants, les antioxydants, les parfums, les conservateurs, les agents conditionneurs, les agents blanchissants destinés à la décoloration des poils et de la peau, les principes actifs destinés à apporter une action traitante vis à vis de la peau ou des cheveux, les filtres solaires, les charges minérales ou les pigments, les particules procurant un effet visuel ou destinées à l'encapsulation d'actifs, les particules exfoliantes, les agents de texture.

Comme exemples de tensioactifs moussants et/ou détergents que l'on peut associer au dit latex inverse auto-inversible tel que défini précédemment dans lesdites compositions (F) et (G), il y a les tensioactifs moussants et/ou détergents anioniques, cationiques, amphotères ou non ioniques.

Parmi les tensioactifs anioniques moussants et/ou détergents que l'on peut associer au dit latex inverse auto-inversible tel que défini précédemment dans lesdites compositions (F) et (G), il y a les sels de métaux alcalins, de métaux alcalino-terreux, d'ammonium, d'amines ou d'aminoalcools d'alkylether sulfates, d'alkyl sulfates, d'alkylamidoéther sulfates, d'alkylaryl polyéthersulfates, de monoglycérides sulfates, d'alphaoléfinesulfonates, de paraffines sulfonates, d'alkyl phosphates, d'alkyléther phosphates, d'alkyl sulfonates, d'alkylamide sulfonates, d'alkylaryl sulfonates, d'alkyl carboxylates, d'alkyl sulfosuccinates, d'alkyléther sulfosuccinates, d'alkylamide sulfosuccinates, d'alkyl sulfoacétates, d'alkyl sarcosinates, d'acyl iséthionates, de N-acyl taurates, d'acyl lactylates, de dérivés N-acylés d'acides aminés, de dérivés N-acylés de peptides, de dérivés N-acylés de protéines, de dérivés N-acylés d'acides gras.

Parmi les tensioactifs amphotères moussants et/ou détergents que l'on peut associer au dit latex inverse auto-inversible tel que défini précédemment dans lesdites compositions (F) et (G), il y a les alkylbétaïnes, les alkylamidobétaïnes, les sultaïnes, les alkylamidoalkylsulfobétaïnes, les dérivés d'imidazolines, les phosphobétaïnes, les amphopolyacétates et les amphopropionates.

Parmi les tensioactifs cationiques moussants et/ou détergents que l'on peut associer au dit latex inverse auto-inversible tel que défini précédemment dans lesdites compositions (F) et (G), il y a particulièrement les dérivés d'ammoniums quaternaires.

Parmi les tensioactifs non ioniques moussants et/ou détergents que l'on peut associer au dit latex inverse auto-inversible tel que défini précédemment dans lesdites compositions (F) et (G), il y a plus particulièrement les alkylpolyglycosides comportant un radical aliphatique, linéaire ou ramifié, saturé ou insaturé, et comportant de 8 à 16 atomes de carbone, comme l'octyl polyglucoside, le décyl polyglucoside, l'undécylényl polyglucoside, le dodécyl polyglucoside, le tétradécyl polyglucoside, l'hexadécyl polyglucoside, le 1-12 dodécanediyl polyglucoside ; les dérivés d'huile de ricin hydrogénée éthoxylés comme le produit commercialisé sous le nom INCI « Peg-40 hydrogenated castor oil » ; les polysorbates comme le Polysorbate 20, le Polysorbate 40, le Polysorbate 60, le Polysorbate 70, le Polysorbate 80, le Polysorbate 85 ; les amides de coprah ; les N-alkylamines.

Comme exemples de tensioactifs épaississants et/ou gélifiants que l'on peut associer au dit latex inverse auto-inversible tel que défini précédemment dans lesdites compositions (F) et (G), il y a les esters gras dàlkylpolyglycosides éventuellement alcoxylés, comme les esters de méthylpolyglucoside éthoxylés tels que le PEG 120 méthyl glucose trioléate et le PEG 120 méthyl glucose dioléate commercialisés respectivement sous les appellations GLUCAMATE^{™} LT et GLUMATE^{™} DOE120 ; les esters gras alcoxylés tels que le PEG 150 pentaérythrytyl tétrastéarate commercialisé sous l'appellation CROTHIX ^{™} DS53, le PEG 55 propylène glycol oléate commercialisé sous l'appellation ANTIL ^{™} 141 ; les carbamates de polyalkylène glycols à chaînes grasses comme le PPG-14 laureth isophoryl dicarbamate commercialisé sous l'appellation ELFACOS ^{™} T211, le PPG-14 palmeth-60 hexyl dicarbamate commercialisé sous l'appellation ELFACOS ^{™} GT2125.

Comme exemples d'agents épaississants et/ou gélifiants que l'on peut associer au dit latex inverse auto-inversible tel que défini précédemment dans lesdites compositions (F) et (G), il y a les copolymères de l'AMPS et d'acrylates d'alkyle dont la chaîne carbonée comprend entre quatre et trente atomes de carbone et plus particulièrement entre dix et trente atomes de carbone, les terpolymère linéaire, branché ou réticulé d'au moins un monomère possédant une fonction acide fort, libre, partiellement salifiée ou totalement salifiée, avec au moins un monomère neutre, et au moins un monomère de formule (XIII) :

CH₂=C(R'₃)-C(=O)-[CH₂-CH₂-O]n'-R' ₄ (XIII)

dans laquelle R'₃ représente un atome d'hydrogène ou un radical méthyle, R4 représente un radical alkyle linéaire ou ramifié comportant de huit à trente atomes de carbone et n' représente un nombre supérieur ou égal à un et inférieur ou égal à cinquante.

Comme exemples d'agents épaississants et/ou gélifiants que l'on peut associer au dit latex inverse auto-inversible tel que défini précédemment dans lesdites compositions (F) et (G), il y a les polysaccharides constitués uniquement d'oses comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1 ).

Comme exemples d'agents épaississants et/ou gélifiants que l'on peut associer au dit latex inverse auto-inversible tel que défini précédemment dans lesdites compositions (F) et (G), il y a les polysaccharides constitués de dérivés d'oses comme les galactanes sulfatés et plus particulièrement les carraghénanes et l'agar les uronanes et plus particulièrement les algines, les alginates et les pectines, les hétéropolymères d'oses et d'acides uroniques et plus particulièrement la gomme xanthane, la gomme gellane, les exsudats de gomme de arabique et de gomme de karaya, les glucosaminoglycanes.

Comme exemples d'agents épaississants et/ou gélifiants que l'on peut associer au dit latex inverse auto-inversible tel que défini précédemment dans lesdites compositions (F) et (G), il y a la cellulose, les dérivés de cellulose comme la méthyl-cellulose, l'éthyl-cellulose l'hydroxypropyl cellulose, les silicates, l'amidon les dérivés hydrophiles de l'amidon, les polyuréthanes.

Comme exemples d'agents stabilisants que l'on peut associer au dit latex inverse auto-inversible tel que défini précédemment dans lesdites compositions (F) et (G), il y a les cires microcristallines, et plus particulièrement l'ozokerite les sels minéraux tels que le chlorure de sodium ou le chlorure de magnésium, les polymères siliconés tels que les copolymères polysiloxane polyalkyl polyéther.

Comme exemples de solvants que l'on peut associer au dit latex inverse auto-inversible tel que défini précédemment dans lesdites compositions (F) et (G), il y a l'eau, les solvants organiques comme le glycérol, le diglycérol, les oligomères du glycérol, l'éthylène glycol, le propylène glycol, le butylène glycol, le 1,3-propanediol, le 1,2-propanediol, l'hexylèneglycol le diéthylèneglycol, le xylitol, l'érythritol le sorbitol, les alcools hydrosolubles tels que l'éthanol, l'isopropanol ou le butanol, les mélanges d'eau et desdits solvants organiques.

Comme exemples d'eaux thermales ou minérales que l'on peut associer au dit latex inverse auto-inversible tel que défini précédemment dans lesdites compositions (F) et (G), il y a les eaux thermales ou minérales ayant une minéralisation d'au moins 300 mg/l, en particulier l'eau d'Avene, l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizieres, l'eau de Neyrac-les-bains, l'eau de Lons le Saunier, l'eau de Rochefort, l'eau de Saint Christau, l'eau des Fumades et l'eau de Tercis-les-bains.

Comme exemples d'agents hydrotropes que l'on peut associer au dit latex inverse auto-inversible tel que défini précédemment, dans lesdites compositions (F) et (G), il y a les xylènes sulfonates, les cumènes sulfonates, l'hexylpolyglucoside le 2-éthylhexylpolyglucoside, le n-heptylpolyglucoside.

Comme exemples d'agents tensioactifs émulsionnants que l'on peut associer au dit latex inverse auto-inversible tel que défini précédemment dans lesdites compositions (F) et (G), il y a les tensioactifs non ioniques, des tensioactifs anioniques, des tensioactifs cationiques.

Comme exemples de tensioactifs non-ioniques émulsionnants que l'on peut associer au dit latex inverse auto-inversible tel que défini précédemment dans lesdites compositions (F) et (G), il y a les esters d'acides gras et de sorbitol, comme les produits commercialisés sous les appellations MONTANE^{™}40, MONTANE^{™}60, MONTANE^{™}70, MONTANE^{™}80 et MONTANE^{™}85 ; les compositions comprenant du stéarate de glycérol et l'acide stéarique éthoxylé entre 5 moles et 150 moles d'oxyde d'éthylène, comme la composition comprenant de l'acide stéarique éthoxylé à 135 moles d'oxyde d'éthylène et du stéarate de glycérol commercialisée sous l'appellation SIMULSOL^{™} 165 ; les esters de mannitan ; les esters de mannitan éthoxylés ; les esters de sucrose ; les esters de méthylglucoside ; les alkylpolyglycosides comportant un radical aliphatique, linéaire ou ramifié, saturé ou insaturé, et comportant de 14 à 36 atomes de carbone, comme le tétradécyl polyglucoside, l'hexadécyl polyglucoside, l'octadécyl polyglucoside, l'hexadécyl polyxyloside, l'octadécyl polyxyloside, l'eicosyl polyglucoside, le dodécosyl polyglucoside, le 2-octyldodécyl polyxyloside, le 12-hydroxystéaryl polyglucoside ; les compositions d'alcools gras linéaires ou ramifiés, saturés ou insaturés, et comportant de 14 à 36 atomes de carbone, et d'alkylpolyglycosides tels que décrits précédemment, par exemple les compositions commercialisées sous les noms MONTANOV^{™}68, MONTANOV^{™}14, MONTANOV^{™}82, MONTANOV^{™}202, MONTANOV^{™}S, MONTANOV^{™}WO18, MONTANOV^{™}L, FLUIDANOV^{™}20X et EASYNOV^{™}.

Comme exemples de tensioactifs anioniques que l'on peut associer au dit latex inverse auto-inversible tel que défini précédemment dans lesdites compositions (F) et (G), il y a le glycéryl stéarate citrate, le cétéarylsulfate, les savons comme le stéarate de sodium ou le stéarate de triéthanolammonium, les dérivés N-acylés d'acides aminés salifiés, par exemple le stéaroyl glutamate.

Comme exemples de tensioactifs cationiques émulsionnants que l'on peut associer au dit latex inverse auto-inversible tel que défini précédemment dans lesdites compositions (F) et (G), il y a les aminoxydes, le quaternium-82 et les tensioactifs décrits dans la demande de brevet WO96/00719 et principalement ceux dont la chaîne grasse comprend au moins 16 atomes de carbone.

Comme exemples d'agents opacifiants et/ou nacrants que l'on peut associer au dit latex inverse auto-inversible tel que défini précédemment dans lesdites compositions (F) et (G), il y a le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras comportant de 12 à 22 atomes de carbone.

Comme exemples d'agents de texture que l'on peut associer au dit latex inverse auto-inversible tel que défini précédemment dans lesdites compositions (F) et (G), il y a des dérivés N-acylés d'acides aminés, comme la lauroyl lysine commercialisée sous l'appellation AMINOHOPE ^{™}LL, l'octenyl starch succinate commercialisé sous l'appellation DRYFLO ^{™}, le myristyl polyglucoside commercialisé sous l'appellation MONTANOV^{™} 14, les fibres de cellulose, les fibres de coton, les fibres de chitosane, le talc, la séricite, le mica

Comme exemples d'agents déodorants que l'on peut associer au dit latex inverse auto-inversible tel que défini précédemment dans lesdites compositions (F) et (G), il y a les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2 décanediol ; le 1,3 propanediol ; l'acide salicylique ; le bicarbonate de sodium ; les cyclodextrines ; les zéolithes métalliques ; le TRICLOSAN^{™} ; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, Ibctochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples d'huiles que l'on peut associer au dit latex inverse auto-inversible tel que défini précédemment dans lesdites compositions (F) et (G), il y a les huiles minérales telles que l'huile de paraffine, l'huile de vaseline, les isoparaffines ou les huiles blanches minérales ; les huiles d'origine animale, telles que le squalène ou le squalane ; les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes les huiles végétales éthoxylées ; les huiles synthétiques comme les esters d'acides gras tels que le myristate de butyle, le myristate de propyle, le myristate d'isopropyle, le myristate de cétyle, le palmitate d'isopropyle, le palmitate d'octyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les isoalcanes de synthèse comme l'isohexadécane, l'isododécane, les huiles perfluorées ; les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles. Par « huiles », on entend dans la présente demande les composés et/ou les mélanges de composés insolubles dans l'eau, se présentant sous un aspect liquide à une température de 25°C.

Comme exemples de cires que l'on peut associer au dit latex inverse auto-inversible tel que défini précédemment dans lesdites compositions (F) et (G), il y a la cire d'abeille, la cire de carnauba, la cire de candelilla, la cire d'ouricoury la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone ; les cires végétales ; les alcools gras et les acides gras solides à température ambiante ; les glycérides solides à température ambiante. Par « cires », on entend dans la présente demande les composés et/ou les mélanges de composés insolubles dans l'eau, se présentant sous un aspect solide à une température supérieure ou égale à 45°C.

Comme exemples de principes actifs que l'on peut associer au dit latex inverse auto-inversible tel que défini précédemment dans lesdites compositions (F) et (G), il y a les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés) ; les composés montrant une action éclaircissante ou dépigmentante de la peau comme le w-undecelynoyl phénylalanine commercialisé sous l'appelation SEPIWHITE ^{™}MSH, le SEPICALM^{™}VG, le mono ester et/ou le diester de glycérol du w-undecelynoyl phénylalanine, les w-undecelynoyl dipeptides, l'arbutine, l'acide kojique, l'hydroquinone; les composés montrant une action apaisante notamment le SEPICALM^{™} S, làllantoïne et le bisabolol ; les agents anti-inflammatoires ; les composés montrant une action hydratante comme l'urée les hydroxyurées, le glycérol, les polyglycérols, le glycérolglucoside, le diglycérolglucoside, les polyglycérylglucosides, le xylitylplucoside ; les extraits végétaux riches en polyphénols comme les extraits de raisin, les extraits de pin, les extraits de vin, les extraits d'olives ; les composés montrant une action amincissante ou lipolytique comme la caféine ou ses dérivés, IADIPOSLIM ^{™}, ADIPOLESS ^{™}, la fucoxanthine ; les protéines N-acylées ; les peptides N-acylés comme le MATRIXIL^{™} ; les acides aminés N-acylés ; les hydrolysâts partiels de protéines N-acylés ; les acides aminés ; les peptides ; les hydrolysâts totaux de protéines ; les extraits de soja, par exemple la Raffermine^{™} ; les extraits de blé par exemple la TENSINE^{™} ou la GLIADINE^{™} ; les extraits végétaux, tels que les extraits végétaux riches en tanins, les extraits végétaux riches en isoflavones ou les extraits végétaux riches en terpènes ; les extraits d'algues d'eau douce ou marines ; les extraits de plantes marines ; les extraits marins en général comme les coraux ; les cires essentielles ; les extraits bactériens ; les céramides ; les phospholipides ; les composés montrant une action antimicrobienne ou une action purifiante, comme le LIPACIDE^{™} C8G, le LIPACIDE^{™} UG, le SEPICONTROL^{™} A5 ; l'OCTOPIROX^{™} ou le SENSIVA^{™} SC50 ; les composés montrant une propriété énergisante ou stimulante comme le PHYSIOGENYL^{™}, le panthénol et ses dérivés comme le SEPICAP^{™} MP ; les actifs anti-âge comme le SEPILIFT^{™} DPHP, le LIPACIDE^{™} PVB, le SEPIVINOL^{™}, le SEPIVITAL^{™}, le MANOLIVA^{™}, le PHYTO-AGE^{™}, le TIMECODE^{™} ; le SURVICODE^{™} ; les actifs anti-photo vieillissement ; les actifs protecteurs de l'intégrité de la jonction dermo-épidermique ; les actifs augmentant la synthèse des composants de la matrice extracellulaire comme le collagène, les élastines, les glycosaminoglycanes ; les actifs agissant favorablement sur la communication cellulaire chimique comme les cytokines ou physiques comme les intégrines ; les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés) ; les actifs améliorant la microcirculation cutanée, par exemple les veinotoniques ; les actifs drainants ; les actifs à visée décongestionnante comme les extraits de ginko biloba, de lierre, de marron d'inde, de bambou, de ruscus, de petit houx, de *centalla asiatica,* de fucus, de romarin, de saule ; les agents de bronzage ou de brunissement de la peau, par exemple la dihydroxyacétone (DHA), l'érythrulose l'aldéhyde mésotartrique, le glutaraldéhyde, le glycéraldéhyde, l'alloxane, la ninhydrine, les extraits végétaux par exemple les extraits de bois rouges du genre Pterocarpus et du genre Baphia comme le Pteropcarpus santalinus, le Pterocarpus osun, le Pterocarpus soyauxii, le Pterocarpus erinaceus, le Pterocarpus indicus ou le Baphia nitida comme ceux décrits dans la demande de brevet Européen EP 0 971 683 ; les agents connus pour leur action de facilitation et/ou d'accélération du bronzage et/ou du brunissement de la peau humaine, et/ou pour leur action de coloration de la peau humaine, par exemple les caraténoïdes ( et plus particulièrement le beta carotène et le gamma carotène), le produit commercialisé sous le nom de marque « Carrot oil » (Nom INCI : Daucus Carota, helianthus annuus Sunflower oil) par la société Provital, qui contient des caroténoïdes, de la vitamine E et de la vitamine K ; la tyrosine et/ou ses dérivés, connus pour leur effet sur l'accélération du bronzage de la peau humaine en association avec une exposition aux rayonnements ultra-violets, par exemple le produit commercialisé sous le nom de marque « SunTan Accelerator^{™} » par la société Provital qui contient de la tyrosine et des riboflavines (vitamine B), le complexe de tyrosine et de tyrosinase commercialisé sous le nom de marque « Zymo Tan Complex » par la société Zymo Line, le produit commercialisé sous le nom de marque MelanoBronze^{™} (nom INCI : Acetyl Tyrosine, Monk's pepper extract (Vitex Agnus-castus)) par la société Mibelle qui contient de l'acétyl tyrosine, produit commercialisé sous le nom de marque Unipertan VEG-24/242/2002 (nom INCI : butylene glycol and Acetyl Tyrosine and hydrolyzed vegetable protein and Adenosine triphosphate) par la société UNIPEX, le produit commercialisé sous le nom de marque « Try-Excell^{™} » (nom INCI : Oleoyl Tyrosine and Luffa Cylindrica (Seed) Oil and Oleic acid) par la société Sederma qui contient des extraits de pépins de courge (ou huile de Loofah), le produit commercialisé sous le nom de marque «Actibronze^{™} » (nom INCI : hydrolyzed wheat protein and acetyl tyrosine and copper gluconate) par la société Alban Muller, le produit commercialisé sous le nom de marque Tyrostan^{™} (nom INCI : potassium caproyl tyrosine) par la société Synerga, le produit commercialisé sous le nom de marque Tyrosinol (nom INCI : Sorbitan Isostearate, glyceryl oleate, caproyl Tyrosine) par la société Synerga, le produit commercialisé sous le nom de marque InstaBronze^{™} (nom INCI : Dihydroxyacetone and acetyl tyrosine and copper gluconate) commercialisé par la société Alban Muller, le produit commercialisé sous le nom de marque Tyrosilane (nom INCI : méthylsilanol and acétyl tyrosine) par la société Exymol ; les peptides connus pour leur effet dàctivation de la mélanogénèse par exemple le produit commercialisé sous le nom de marque Bronzing SF Peptide powder (nom INCI : Dextran and Octapeptide-5) par la société Infinitec Activos, le produit commercialisé sous le nom de marque Melitane (nom INCI : Glycerin and Aqua and Dextran and Acetyl hexapeptide-1) comprenant l'acétyl hexapeptide-1 connu pour son action agoniste de l'alpha-MSH, le produit commercialisé sous le nom de marque Melatimes Solutions^{™} (nom INCI : Butylene glycol ,

Palmitoyl Tripeptide-40) par la société LIPOTEC, les sucres et les dérivés de sucres par exemple le produit commercialisé sous le nom de marque Tanositol^{™} (nom INCI : inositol) par la société Provital, le produit commercialisé sous le nom de marque Thalitan^{™} (ou Phycosaccharide^{™} AG) par la société CODIF international (nom INCI : Aqua and Hydrolyzed algin (Laminaria Digitata) and magnesium sulfate and manganese sulfate) contenant un oligosaccharide d'origine marine (acide guluronique et acide mannuronique chélatés avec les ions magnésium et manganèse), le produit commercialisé sous le nom de marque Melactiva^{™} (nom INCI : Maltodextrin, Mucuna Pruriens Seed extract) par la société Alban Muller, les composés riches en flavonoïdes par exemple le produit commercialisé sous le nom de marque « Biotanning » (nom INCI : Hydrolyzed citrus Aurantium dulcis fruit extract) par la société Silab et connu pour être riche en flavonoides de citron (de type hespéridines) ; les agents destinés au traitement des cheveux et/ou des poils, par exemple des agents protecteurs des mélanocytes du follicule pileux, destinés à protéger lesdits mélanocytes contre les agents cytotoxiques responsables de la sénescence et/ou de l'apoptose desdits mélanocytes, tels que les agents mimétiques de l'activité de la DOPAchrome tautomérase choisis parmi ceux décrits dans la demande de brevet européen publiée sous le numéro EP1515688 A2, les molécules synthétiques mimétiques de la SOD par exemples les complexes de manganèse, des composés antioxydants par exemple les dérivés de cyclodextrine, des composés silicés dérivés d'acide ascorbique, de la pyrrolidone carboxylate de lysine ou d'arginine, des associations de mono- et diester d'acide cinnamique et de vitamine C, et plus généralement ceux cités dans la demande de brevet européen publiée sous le numéro EP 1 515 688 A2.

Comme exemples d'agents antioxydants que l'on peut associer au dit latex inverse auto-inversible tel que défini précédemment dans lesdites compositions (F) et (G), il y a l'EDTA et ses sels, l'acide citrique, l'acide tartarique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE GL 47S commercialisé par la société Akzo Nobel sous le nom INCI : Tetrasodium Glutamate Diacetate.

Comme exemples de filtres solaires que l'on peut associer au dit latex inverse auto-inversible tel que défini précédemment dans lesdites compositions (F) et (G), il y a tous ceux figurant dans la directive cosmétique 76/768/CEE modifiée annexe VII.

Parmi les filtres organiques solaires que l'on peut associer au dit latex inverse auto-inversible tel que défini précédemment dans lesdites compositions (F) et (G), il y a la famille des dérivés de l'acide benzoïque comme les acides para-aminobenzoïques (PABA), notamment les esters de monoglycérol de PABA, les esters éthyliques de N,N25 propoxy PABA, les esters éthyliques de N,N-diéthoxy PABA, les esters éthyliques de N,Ndiméthyl PABA, les esters méthyliques de N,N-diméthyl PABA, les esters butyliques de N,Ndiméthyl PABA; la famille des dérivés de l'acide anthranilique comme l'homomenthyl-N-acétyl anthranilate ; la famille des dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle ; la famille des dérivés de l'acide cinnamique comme le cinnamate d'éthylhexyle, le cinnamate d'éthyl-4-isopropyle, le cinnamate de méthyl- 2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de pméthoxy 2-éthylhexyle), le cinnamate de p-méthoxy 2-éthoxyéthyle, le p-cinnamate de méthoxycyclohexyle, le cinnamate d'éthyl-α-cyano-β-phényle, le cinnamate de 2-éthylhexyl-α-cyano-β-phényle, le cinnamate de diparaméthoxy mono-2-éthylhexanoyl de glycéryle ; la famille des dérivés de la benzophénone comme la 2,4-dihydroxybenzophénone, la 2,2'-dihydroxy-4-méthoxybenzophénone, la 2,2',4,4'-tétrahydroxybenzophénone, la 2-hydroxy-4-méthoxybenzophénone, la 2-hydroxy-4-méthoxy-4'-méthylbenzophénone, la 2-hydroxy-4-méthoxybenzophénone-5-sulfonate, la 4-phénylbenzophénone, le 2-éthylhexyl-4'-phénylbenzophénone-2-5 carboxylate, la 2-hydroxy-4-n-octyloxybenzophénone, la 4-hydroxy-3-carboxybenzophénone ; le 3-(4'-méthylbenzylidène)-d,l-camphre, le 3 (benzylidène)-d,lcamphre, le benzalkonium méthosulfate camphre ; l'acide urocanique, l'urocanate d'éthyle ; la famille des dérivés de l'acide sulfonique comme l'acide sulfonique 2-phénylbenzimidazole-5 et ses sels ; la famille des dérivés de la triazine comme l'hydroxyphényl triazine, l'éthylhexyloxyhydroxyphényl-4-méthoxyphényltriazine, le 2,4,6-trianillino-(p-carbo-2-éthylhexyl-1'oxy)-1,3,5-triazine, le 4,4-((6-(((1,1-diméthyléthyl) amino)carbonyl)phenyl)amino)-1,3,5-triazine-2,4-diyl diimino) bis-(2-éthylhexyl) ester de l'acide benzoïque, le 2-phényl-5-méthylbenzoxazole, le 2,2'-hydroxy-5-méthylphénylbenzotriazole, le 2-(2'-hydroxy-5'-t-octylphényl)benzotriazole, le 2-(2'-hydroxy-5'-méthyphényl)benzotriazole; la dibenzazine; le dianisoylméthane, le 4-méthoxy-4"-tbutylbenzoylméthane ; la 5-(3,3-diméthyl-2-norbornylidène)-3-pentan-2-one ; la famille des dérivés du diphénylacrylate comme le 2-éthylhexyl-2-cyano-3,3-diphényl-2-propènoate, l'éthyl-2-cyano-3,3-diphényl-2-propènoate; la famille des polysiloxanes comme le malonate de benzylidène siloxane.

Parmi les filtres inorganiques solaires, également appelés "écrans minéraux", que l'on peut associer au dit latex inverse auto-inversible tel que défini précédemment dans lesdites compositions (F) et (G), il y a les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

L'invention a enfin pour objet lùtilisation dune composition (C ₑ) comme agent inverseur d'un latex inverse d'un polyélectrolyte anionique réticulé (P) comprenant pour 100% molaire :
(a₁) - d'une proportion supérieure ou égale à 30% molaire et inférieure ou égale à 100% molaire, d'unités monomériques issues de l'acide 2-méthyl 2-[(1-oxo 2-propènyl) amino] 1-propanesulfonique sous forme acide libre ou partiellement ou totalement salifiée ;
(a₂) - optionnellement d'une proportion supérieure à 0% molaire et inférieure ou égale à 70% molaire, d'unités monomériques issues d'au moins un monomère choisi parmi les éléments du groupe constitué par l'acide acrylique, l'acide méthacrylique, l'acide 2-carboxyéthyl acrylique, l'acide itaconique, l'acide maléique, l'acide 3-méthyl 3-[(1-oxo 2-propènyl) amino] butanoïque, la fonction carboxylique desdits monomères étant sous forme acide libre, partiellement salifiée ou totalement salifiée et/ou parmi les éléments du groupe constitué par l'acrylate de (2-hydroxy éthyle), l'acrylate de (2,3-dihydroxy propyle), le méthacrylate de (2-hydroxy éthyle), le méthacrylate de (2,3-dihydroxypropyle), ou le vinyl pyrrolidone ;
(a₃) - d'une proportion supérieure à 0% molaire et inférieure ou égale à 1% molaire d'unités monomériques issues d'au moins un monomère de réticulation (AR) diéthylénique ou polyéthylénique;
   la somme des dites proportions molaires en unités monomériques selon a₁), a₂) et a₃) étant égale à 100% molaire;

ledit latex inverse étant une émulsion de type eau-dans-huile (E) comprenant pour 100% de sa masse :
   **a)** - de 10% massique à 90% massique, dudit polyélectrolyte anionique réticulé (P) ;
   **b) -** De 5% massique à 50% massique, d'une phase grasse constituée d'au moins une huile (H),
   **c)** - De 1% massique à 50% massique d'eau, et
   **d) -** De 0,5% massique à 10% massique d'un système émulsionnant de type eau-dans-huile (S₁).
      la somme des proportions massiques en composés selon a), b), c) et d) étant égale à 100% massique,
ladite composition (Cₑ) comprenant pour 100% de sa masse :
   **e₁)** - De 10% massique à 60% massique, plus particulièrement de 15% massique à 60% massique et encore plus particulièrement de 15% massique à 50% massique, d'au un composé de formule (I) :

      HO-[CH₂-CH(OH)-CH₂-O]ₙ-H (I)

      dans laquelle n représente un nombre entier supérieur ou égal à un et inférieur ou égal à quinze ;
   **e₂)** - De 40% massique à 90 % massique, plus particulièrement de 40% massique à 85% massique et encore plus particulièrement de 50% massique à 85% massique, d'au moins un composé de formule (II) :

      R₁-(C=O)-[O-CH₂-CH(OH)-CH₂]ₚ-OH (II),

      dans laquelle p, différent ou identique à n, représente un nombre entier supérieur ou égal à un et inférieur ou égal à quinze et le groupe R₁-(C=O)- représente un radical aliphatique saturé ou insaturé, linéaire ou ramifié, comportant de six à vingt-deux atomes de carbone, et optionnellement
   **e₃)** - Jusquà 30% massique 0% massique à 30 % massique, plus particulièrement de 0% massique à 25% massique et plus particulièrement de 0% massique à 20% massique d'au moins une composition (C ₁₁) représentée par la formule (III) :

      HO-[CH₂-CHOH-CH₂-O-]_{q}-(G)ᵣ-H (III),

      formule (III) dans laquelle q, différent ou identique à n, représente un nombre entier supérieur ou égal à un et inférieur ou égal à 3, G représente le reste d'un sucre réducteur et r représente un nombre décimal supérieur ou égal à 1,05 et inférieur ou égal à 5,00, ladite composition (C₁₁) consistant en un mélange des composés de formules (III₁), (III₂), (III₃), (III₄) et (III₅) :

      HO-[CH₂-CHOH-CH₂-O-]_{q} -O-(G)₁-H (III₁),

      HO-[CH₂-CHOH-CH₂-O-]_{q} -O-(G)₂-H (III₂),

      HO-[CH₂-CHOH-CH₂-O-]_{q} -O-(G)₃-H (III₃),

      HO-[CH₂-CHOH-CH₂-O-]_{q} -O-(G)₄-H (III₄),

      HO-[CH₂-CHOH-CH₂-O-]_{q} -O-(G)₅-H (III₅),
en des proportions molaires en dits composés de formules (III₁), (III₂), (III₃), (III₄) et (III₅) respectivement égales à a₁, a₂, a₃, a₄ et a₅, telles que la somme a₁+ a₂ + a₃ + a4 + a₅ est égale à un, et que la somme a₁ + 2a₂ + 3a₃ + 4a₄ + 5a₅ est égale à r ;
la somme des proportions massiques en composés selon e₁), e₂) et e₃) étant égale à 100% massique.

Selon un aspect particulier, la composition (Ce) est caractérisée en ce que dans la formule (I) n représente un nombre entier supérieur ou égal à un et inférieur ou égal à dix et dans la formule (II) p, différent de n, représente un nombre entier supérieur ou égal à un et inférieur ou égal à dix.

Selon cet aspect particulier, la composition (Ce) est plus particulièrement caractérisée en ce que dans la formule (I) telle que définie précédemment, n est égal à 1 ou à 6 et dans la formule (II) p est égal à 6 ou à 10.

Selon cet aspect particulier, la composition (Cₑ) est aussi **caractérisée en ce que** dans les formules (I) et (II) telle que définies précédemment, n et p sont identiques et représentent chacun un nombre entier supérieur ou égal à 1 et inférieur ou égal à 10 et notamment supérieur ou égal à 4 et inférieur ou égal à 8.

Selon cet aspect particulier, la composition (Ce) est plus particulièrement caractérisée en ce que dans les formules (I) et (II) telles que définies, n et p sont égaux à 6.

Selon un autre aspect particulier de la présente invention, dans la formule (II), le groupe R₁-(C=O)- est choisi parmi les éléments du groupe constitué par les radicaux octanoyle, décanoyle, w-undécylènoyle, dodécanoyle, tétradécanoyle, hexadécanoyle, octadécanoyle, 9-octadécènoyle ou 9,12-octadécadiènoyle ; il s'agit plus particulièrement du radical dodécanoyle.

Selon un aspect particulier, la composition (Cₑ) consiste en, pour 100% de sa masse :
**e₁)** - De 10% massique à 60% massique d'au moins un composé de formule (I) telle que définie précédemment et
**e₂)** - De 40% massique à 90 % massique d'au moins un composé de formule (II) telle que définie précédemment.

Selon un aspect particulier, dans la formule (III) telle que définie précédemment, q est égal à un, G représente le reste du glucose et r représente un nombre décimal supérieur ou égal à 1,05 et inférieur ou égal à 2,5.

Selon un autre aspect particulier de la présente invention, la composition (Cₑ) telle que définie précédemment est **caractérisée en ce qu**'elle consiste en, pour 100% de sa masse :
**e₁)** - De 5% massique à 15% massique d'au moins un composé de formule (I) telle que définie précédemment,
**e₂)** - De 60% massique à 80 % massique d'au moins un composé de formule (II) telle que définie précédemment, et
**e₃)** - De 5% à 15% massique d'au moins une composition (C ₁₁) représentée par la formule (III) telle que définie précédemment.

Par sucre réducteur, on désigne dans la formule (III), les dérivés saccharidiques tels que définis précédemment et G représente, dans la formule (III) telle que définie précédemment, le reste d'un sucre réducteur choisi parmi les restes du glucose, du dextrose, du saccharose, du fructose, de l'idose, du gulose, du galactose, du maltose, de l'isomaltose du maltotriose, du lactose, du cellobiose, du mannose, du ribose, du xylose, de l'arabinose, du lyxose, de de l'allose, de l'altrose du dextrane ou du tallose, et plus particulièrement parmi les restes du glucose, du xylose et de l'arabinose.

La formule (III) représentant la composition (C₁₁), consiste en un mélange de composés représentés par les formules (III₁), (III₂), (III₃), (III₄) et (III₅) tel que défini précédemment. Selon un aspect plus particulier de la présente invention, dans la formule (III) représentant la composition (C₁₁) telle que définie précédemment, r représente un nombre décimal supérieur ou égal à 1,05 et inférieur ou égal à 3, plus particulièrement supérieur ou égal à 1,15 et inférieur ou égal à 2,5.

Selon un aspect plus particulier, dans la formule (III), q est égal à un, G représente le reste du glucose et r représente un nombre décimal supérieur ou égal à 1,05 et inférieur ou égal à 2,5.

Selon un autre aspect particulier, dans la formule (I), n représente un nombre entier supérieur ou égal à un et inférieur ou égal à dix, dans la formule (II) p, identique ou différent de n, représente un nombre entier supérieur ou égal à un et inférieur ou égal à dix et le groupe R₁-(C=O)- est choisi parmi les radicaux octanoyle, décanoyle, w-undécylènoyle, dodécanoyle, tétradécanoyle, hexadécanoyle, octadécanoyle, 9-octadécènoyle ou 9,12-octadécadiènoyle, et dans la formule (III), q est égal à un, G représente le reste du glucose et r représente un nombre décimal supérieur ou égal à 1,05 et inférieur ou égal à 2,5.

Selon un aspect tout particulier, dans la formule (I) n est égal à un, dans la formule (II) p est égal à 10 et le groupe R₁-(C=O)- est le radical dodécanoyle et dans la formule (III), q est égal à un, G représente le reste du glucose et r représente un nombre décimal supérieur ou égal à 1,05 et inférieur ou égal à 2,5.

La composition (C₁₁) optionnellement comprise dans la composition (Ce) est préparée selon un procédé comprenant les étapes suivantes :
- Une étape A) de réaction, dans les proportions souhaitées, d'un sucre réducteur de formule (XI) ou un mélange de sucres réducteurs de formule (XI) :

   HO-G-H (XI)

   dans laquelle G représente le reste d'un sucre réducteur, avec un excès molaire dùn composé de formule (XII) ou dùn mélange de composés de formule (XII) :

   HO-[CH₂-CHOH-CH₂-O-]_{q}H (XII),

   formule (XII) pour laquelle q est tel que défini précédemment pour la formule (III), pour former un mélange de composés de formule (III) tels que définis précédemment et un excès dudit composé de formule (XII).

L'étape A) du procédé tel que défini précédemment, peut être complétée, si nécessaire ou si désirée, par des opérations ultérieures de neutralisation, par exemple à la soude ou à la potasse, et/ou de filtration, et/ou de décoloration, et/ou d'élimination du polyol résiduel par exemple par extraction sélective au moyen d'un milieu solvant adapté.

L'étape A) est généralement mise en œuvre dans un réacteur en présence d'un système catalytique acide, en maîtrisant le rapport stœchiométrique entre les deux réactants, et plus particulièrement en introduisant un excès molaire du mélange d'alcools de formule (II), sous agitation mécanique dans des conditions de température et de vide partiel prédéterminées, par exemple à une température comprise entre 70°C et 130°C et sous un vide partiel compris entre 300mbar (3.10⁴Pa) et 20mbar (2.10³Pa). Par système catalytique acide, on désigne les acides forts comme l'acide sulfurique, l'acide chlorhydrique, l'acide phosphorique, l'acide nitrique, l'acide hypophosphoreux, làcide méthanesulfonique, l'acide para-toluène sulfonique, l'acide trifluorométhanesulfonique, ou les résines échangeuses d'ions.

Les exemples suivants illustrent l'invention, sans toutefois la limiter.

### I- Préparation de compositions tensioactives selon l'invention et comparatives

### I_{A}- Préparation d'une composition comprenant du glycéryl polyglucoside et du glycérol

On introduit 650 grammes de glycérol, soit 5 équivalents molaires, dans un réacteur en verre à double enveloppe, dans laquelle circule un fluide caloporteur, et muni d'une agitation efficace. Le glycérol est porté à une température d'environ 100°C.

423,9 grammes, soit un équivalent molaire, de glucose sont alors ajoutés progressivement au milieu réactionnel pour permettre sa dispersion homogène. On ajoute au mélange ainsi obtenu un système catalytique acide constitué de 0,51 gramme d'acide sulfurique à 98%.

Le milieu réactionnel est placé sous un vide partiel à 30 mbars, et maintenu à une température de 100°C-105°C pendant une durée de quatre heures, avec évacuation de l'eau formée au moyen d'un montage de distillation.

Le milieu réactionnel est ensuite refroidi à 95°C-100°C et neutralisé par ajout de soude à 30%, pour porter le pH d'une solution à 1% de ce mélange à une valeur d'environ 7,0.

Le mélange réactionnel est vidangé pour obtenir la composition référencée (E_{IB}).

Les caractéristiques analytiques de la composition (E_{IB}) ainsi obtenue sont les suivantes :
Aspect (visuel) : liquide limpide ;
pH solution à 1% : 6,8 ;
Glycérol résiduel : 55,1% ;
Glucose résiduel : < 1% ;
Glycéryl polyglucosides : 44,7%

### I_{B}- Préparation de la composition (EM₂) à base de laurate de décaglycérol (EM₁) et d'hexaglycérol

On introduit dans un réacteur en verre à double enveloppe, dans laquelle circule un fluide caloporteur, et muni d'une agitation mécanique efficace 71,5 grammes de monolaurate de décaglycérol commercialisé sous le nom de marque DECAGLYN 1-L (ci-après désignée par le terme « Composition (EM₁) »), et 28,5 grammes de polyglycérol-6 (commercialisé sous le nom de marque Polyglycerol6^{™} par la société SPIGA), à une température de 35°C sous une agitation mécanique de type ancre à une vitesse de 80 tours/minute. Après mélange dans de telles conditions pendant 30 minutes, le mélange est vidangé pour obtenir la composition (EM₂).

### I_{c}- Préparation de la composition (EM₃) à base de laurate de décaglycérol (EM₁) et de décaglycérol

On introduit dans un réacteur en verre à double enveloppe, dans laquelle circule un fluide caloporteur, et muni d'une agitation mécanique efficace 71,5 grammes de monolaurate de décaglycérol commercialisé sous le nom de marque DECAGLYN 1-L (ci-après désignée par le terme « Composition **(EM₁)** »), et 28,5 grammes de polyglycérol-10 (commercialisé sous le nom de marque Polyglycerin 10^{™}), à une température de 35°C sous une agitation mécanique de type ancre à une vitesse de 80 tours/minute.

Après mélange dans de telles conditions pendant 30 minutes, le mélange est vidangé pour obtenir la composition **(EM₃).**

### I_{D}- Préparation de la composition (EM₄) à base de laurate de décaglycérol (EM₁), de glycéryl polyglucoside et de glycérol (E_{IB})

On introduit dans un réacteur en verre à double enveloppe, dans laquelle circule un fluide caloporteur, et muni d'une agitation mécanique efficace 71,5 grammes de monolaurate de décaglycérol commercialisé sous le nom de marque DECAGLYN 1-L (ci-après désignée par le terme « Composition (EM₁) »), et 28,5 grammes de la composition (E_{IB}), dont la préparation est décrite ci-dessus, à une température de 35°C sous une agitation mécanique de type ancre à une vitesse de 80 tours/minute. Après mélange dans de telles conditions pendant 30 minutes, le mélange est vidangé pour obtenir la composition **(EM₄).**

### I_{E}- Préparation de la composition (EM₅) à base de laurate de décaglycérol (EM₁) et de glycérol

On introduit dans un réacteur en verre à double enveloppe, dans laquelle circule un fluide caloporteur, et muni d'une agitation mécanique efficace 71,5 grammes de monolaurate de décaglycérol commercialisé sous le nom de marque DECAGLYN 1-L (ci-après désignée par le terme « Composition (EM₁) »), et 28,5 grammes de glycérol, à une température de 35°C sous une agitation mécanique de type ancre à une vitesse de 80 tours/minute. Après mélange dans de telles conditions pendant 30 minutes, le mélange est vidangé pour obtenir la composition **(EM₅).**

Les caractéristiques analytiques des compositions (EM₁), (EM₂), (EM₃), (EM₄), (EM₅) et (EM₆) sont consignées dans le tableau 1 ci-dessous.

**Tableau 1**

| | Composition émulsionnante | | | | |
|---|---|---|---|---|---|
| | (EM₁) | (EM₂) | (EM₃) | (EM₄) | (EM₅) |
| Proportions en constituants (% massique) | | | | | |
| Monolaurate de decaglycérol | 100% | 71,5% | 71,5% | 71,5% | 71,5% |
| Proportion massique d'héxaglycérol | 0% | 28,5 | 0% | 0% | 0% |
| Proportion massique de décaglycérol | 0% | 0% | 28,5 | 0% | 0% |
| Proportion massique de glycéryl polyglucoside | 0% | 0% | 0% | 12,8% | 0% |
| Proportion massique de glycérol | 0% | 0% | 0% | 15,7% | 28,5 |

### II - Préparation et évaluation de latex inverses auto-inversibles d'un copolymère réticulé du sel de sodium de l'acide 2-méthyl-[(1-oxo-2-propènyl) amino] 1-propanesulfonique et de l'acide acrylique partiellement salifié.

On prépare une phase aqueuse en versant successivement dans un bécher et sous agitation 75,4 grammes d'acide acrylique glacial, 577,5 grammes d'une solution aqueuse à 55% de sel de sodium d'acide 2-méthyl-[(1-oxo-2-propenyl) amino] 1-propanesulfonique, 42,5 grammes d'une solution aqueuse à 48% en masse d'hydroxyde de sodium, 0,45 gramme d'une solution aqueuse commerciale à 40% en masse de diéthylènetriamine penta-acétate de sodium et 0,167 gramme de méthylène bis(acrylamide). Le pH de cette phase aqueuse est ensuite ajusté à 5,5.

On prépare indépendamment une phase organique en mélangeant 208 grammes du mélange d'alcanes commercialisé sous le nom de marque Emogreen ^{™}L15, 14 grammes de Montane^{™} 80, 9,5 grammes de Montane^{™} 70 et 0,2 gramme d'azo bis(isobutyronitryle) (AIBN).

La phase aqueuse préparée est ensuite ajoutée progressivement sur la phase huileuse puis dispersée à l'aide d'un rotor stator type Ultra Turrax commercialisé par la société lka.

Lémulsion obtenue est transférée dans un réacteur pour être soumise à un barbotage dàzote pour éliminer l'oxygène et refroidie à environ 5-6 °C. 5 cm ³ d'une solution à 0,42% en masse d'hydroperoxyde de cumène dans l'Emogreen ^{™}L15 sont ajoutés à l'émulsion maintenue sous agitation, puis une solution aqueuse à 0,1% en masse de métabisulfite de sodium est introduite progressivement à un débit de 0,5 cm³ par minute pour initier la réaction de polymérisation. La température du milieu augmente jusqu'à atteindre un palier. Le milieu réactionnel est ensuite chauffé à 85°C pendant une heure puis l'ensemble est refroidi jusquà environ 35°C pour obtenir le mélange noté (M₂).

Le mélange (M₂) précédemment obtenu est fractionné en différentes portions auxquelles sont ajoutées les différentes compositions tensioactives (EM₁), (EM₂), (EM₃), (EM₄) et (EM₅), telles que décrites ci-dessus, préalablement chauffées à 60°C, dans des proportions massiques telles qu'indiquées dans le tableau 2 ci-dessous.

Les latex inverses auto-inversibles résultant de ces mélanges sont respectivement notés (LI₁), (LI₂), (LI₃), (LI₄) et (LI₅), et sont évalués par l'observation de leur aspect à 25°C, par la vitesse d'inversion lors de la préparation d'un gel aqueux à 2% massique de latex inverse auto-inversible, par la viscosité de ce gel aqueux à 2% massique d'un latex inverse auto-inversible.

La méthode d'évaluation de la durée d'inversion des latex inverses auto-inversibles consiste à introduire dans un bécher de 2 litres, la quantité d'eau nécessaire à la préparation d'un gel aqueux de 800 grammes. On place vers le fond bécher une hélice d'agitateur mécanique de type Turbotest ^{™} version 2004 commercialisé par la société VMI, connectée à un moteur. L'agitation est démarrée à une vitesse de 900 tours /minute et la quantité nécessaire de latex inverse auto-inversible à évaluer est introduite dans le bécher sous agitation. l'agitation crée un vortex qui disparaît lorsque le polymère s'inverse et le gel se forme. La durée d'inversion mesurée en secondes, des latex inverses auto-inversibles correspond au temps écoulé entre le début de l'ajout du latex inverse auto-inversible testé et la disparition du vortex, conduisant à l'obtention d'un gel lisse, exempt de grumeaux. Cette évaluation est réalisée à l'issue de la fabrication des latex inverses testés (t = 0), puis après une période de stockage de 3 mois à 25°C (t = 3mois). Les résultats obtenus sont consignés dans le tableau 2 ci-dessous. La viscosité d'un gel aqueux à 2% massique de latex inverse auto-inversible (µ), est mesurée à t = 0 puis à t = 3 mois, au moyen d'un viscosimètre Brookfield RVT (Mobile 6 Vitesse 5). De même, l'aspect du latex inverse auto- inversible est évaluée visuellement à t = 0.

**Tableau 2**

| | Latex inverses auto-inversibles | | | | |
|---|---|---|---|---|---|
| | (LI₁) | (LI₂) | (LI₃) | (LI₄) | (LI₅) |
| | Référence de la composition tensioactive testée | | | | |
| | (EM₁) | (EM₂) | (EM₃) | (EM₄) | (EM₅₎ |
| Quantité testée (EMi)/(Lli) (% massique) | 5% | 7% | 7% | 7% | 7% |

| | Mesures à t = 0 | | | | |
|---|---|---|---|---|---|
| µ (en mPas) | 120.000 | 116.000 | 104.000 | 98.000 | 104.000 |
| Durée d'inversion | 130s | 38s | 38s | 60s | 16s |
| Aspect du latex auto-inversible à 25°C | Ell* | Ell* | Ell* | Ell* | Ell* |

| | Mesures à T = 3 mois (3M) | | | | |
|---|---|---|---|---|---|
| µ (en mPas) | 97 000 | 98 000 | 100 000 | 94 000 | 95 000 |
| Durée d'inversion | 151 s | 55 s | 78 s | 78 s | 17 s |

| | | | | | |
|---|---|---|---|---|---|
| Ell* : Emulsion laiteuse liquide | | | | | |

Les latex inverses auto-inversibles (LI₂), (LI₃), (LI₄) et (LI₅) selon l'invention, et exempts de dérivés alkoxylés et plus particulièrement éthoxylés, permettent d'obtenir des gels lisses, avec une durée d'inversion largement inférieure à celle observée pour le latex inverse auto-inversible (LI₁), ne comprenant que le seul monolaurate de décaglycérol comme constituant du système tensioactif inverseur, tout en conservant des propriétés épaississantes excellentes. De plus, ils se caractérisent par une meilleure reproductibilité de la vitesse d'inversion et des propriétés épaississantes après trois mois de stockage que pour le latex inverse auto-inversible comparatif (LI₁).

### III - Préparation et évaluation de latex inverses auto-inversibles d'un copolymère réticulé du sel de sodium de l'acide 2-méthyl-[(1-oxo-2-propènyl) amino] 1-propanesulfonique et de l'acide acrylique partiellement salifié.

On reproduit l'exemple II, décrit ci-dessus, en remplaçant les 208 grammes d'Emogreen ^{™}L15 par 208 grammes d'isohexadécane pour obtenir le mélange noté (M₃), qui est fractionné en différentes portions auxquelles sont ajoutées les différentes compositions tensioactives (EM₁), (EM₂) et (EM₃), telles que décrites ci-dessus, préalablement chauffées à 60 °C, dans des proportions massiques telles qui'ndiquées dans le tableau 3 ci-dessous. Les latex inverses auto-inversibles résultant de ces mélanges sont respectivement notés (LI₆), (LI₇) et (LI₈), et sont évalués par l'observation de leur aspect à 25°C, par la vitesse d'inversion lors de la préparation d'un gel aqueux à 2% massique de latex inverse auto-inversible (dont la méthode est décrite ci-dessus), par la viscosité de ce gel aqueux à 2% massique d'un latex inverse auto-inversible (µ ; viscosimètre Brookfield RVT (Mobile 6 Vitesse 5)). Cette évaluation est réalisée à l'issue de la fabrication des latex inverses testés (t = 0), puis après une période de stockage de 3 mois à 25°C (t = 3 mois).

Les résultats obtenus sont consignés dans le tableau 3 ci-dessous.

**Tableau 3**

| | Latex inverses auto-inversibles | | |
|---|---|---|---|
| | (LI₆) | (LI₇) | (LI₈) |
| | Composition tensioactive testée | | |
| | (EM₁) | (EM₂) | (EM₃) |
| Quantité testée (EMi)/(Lli) (% massique) | 5% | 7% | 7% |

| | Mesures à t = 0 | | |
|---|---|---|---|
| µ (en mPas) | 108 000 | 106 000 | 110 000 |
| Durée di'nversion | 125 s | 60 s | 25 s |
| Aspect du latex auto-inversible à 25°C | Ell* | Ell* | Ell* |

| | Mesures à t = 3 mois | | |
|---|---|---|---|
| µ (en mPas) | 97 000 | 102 000 | 105 000 |
| Durée di'nversion | 155 s | 70 s | 72 s |

| | | | |
|---|---|---|---|
| Ell* : Emulsion laiteuse liquide | | | |

Les latex inverses auto-inversibles (LI₇) et (LI₈) selon l'invention, et exempts de dérivés alkoxylés et plus particulièrement éthoxylés, permettent d'obtenir des gels lisses, avec une durée d'inversion largement inférieure à celle observée pour le latex inverse auto-inversible (LI₆), ne comprenant que le seul monolaurate de décaglycérol comme constituant du système tensioactif inverseur, tout en conservant des propriétés épaississantes excellentes. De plus, ils se caractérisent par une meilleure reproductibilité de la vitesse d'inversion et des propriétés épaississantes après trois mois de stockage que pour le latex inverse auto-inversible comparatif (LI₁).

### IV : Formulations cosmétiques illustratives

Dans les formulations suivantes, les pourcentages sont exprimés en pourcentage massique pour 100% de la masse de la formulation.

### Exemple IV-1 : Crème de soin

| | |
|---|---|
| Cyclométhicone : | 10% |
| Latex inverse auto-inversible (LI₂) : | 0,8% |
| Montanov^{™}68 : | 2% |
| Alcool stéarylique : | 1% |
| Alcool stéarique : | 0,5% |
| Conservateur : | 0,65% |
| Lysine : | 0,025% |
| EDTA (sel disodique) : | 0,05% |
| Gomme de xanthane : | 0,2% |
| Glycérine : | 3% |
| Eau : q.s.p. | 100% |

### Exemple IV-2 : Lait solaire

| FORMULE | |
|---|---|
| A Montanov^{™}68 : | 3,0% |
| Huile de sésame : | 5,0% |
| PARSOL^{™} MCX : | 5,0% |
| Carraghénane λ : | 0,10% |
| B Eau : | q.s.p. 100% |
| C Latex inverse auto-inversible (LI₃) : | 0,80% |
| D Parfum : | q.s. |
| Conservateur : | q.s. |

### MODE OPERATOIRE

Emulsionner B dans A à 60°C puis ajouter C vers 60°C, puis D vers 30°C et ajuster le pH si nécessaire.

### Exemple IV-3: Lait corporel

| | |
|---|---|
| Montanov^{™}202 : | 3,5% |
| LANOL^{™} 37T : | 8,0% |
| SOLAGUM^{™} L : | 0,05% |
| Eau : | q.s.p. 100% |
| Benzophénone-3 : | 2,0% |
| Diméthicone 350cPs : | 0,05% |
| Latex inverse auto-inversible (LI₂) : | 2,5% |
| Conservateur : | 0,2% |
| Parfum : | 0,4% |

### Exemple IV-4: Emulsion démaquillante à l'huile d'amandes douces

| | |
|---|---|
| Montanov^{™}202 : | 5% |
| Huile d'amandes douces : | 5% |
| Eau : | q.s.p. 100% |
| Latex inverse auto-inversible (LI₂) : | 0,3% |
| Glycérine : | 5% |
| Conservateur : | 0,2% |
| Parfum : | 0,3% |

### Exemple IV-5: Crème hydratante pour peaux grasses

| | |
|---|---|
| Montanov^{™}68 : | 5% |
| Cétylstéaryloctanoate : | 8% |
| Palmitate d'octyle : | 2% |
| Eau : | q.s.p. 100% |
| Latex inverse auto-inversible (LI₄) : | 2,6% |
| MICROPEARL^{™} M100 : | 3,0% |
| Mucopolysaccharides : | 5% |
| SEPICIDE^{™} HB : | 0,8% |
| Parfum : | 0,3% |

### Exemple IV-6: Lait démaquillant

| | |
|---|---|
| Montanov^{™}68 : | 3% |
| PRIMOL^{™} 352 : | 8,0% |
| Huile d'amandes douces : | 2% |
| Eau : | q.s.p. 100% |
| Latex inverse auto-inversible (LI₂) : | 0,8% |
| Conservateur : | 0,2% |

### Exemple IV-7: Lait solaire

| | |
|---|---|
| Montanov^{™} L : | 3,5% |
| LANOL^{™} 37T : | 10,0% |
| PARSOL^{™} MCX : | 5,0% |
| EUSOLEX^{™} 4360 : | 2,0% |
| Eau : | q.s.p. 100% |
| Latex inverse auto-inversible (LI₂) : | 1,8% |
| Conservateur : | 0,2% |
| Parfum : | 0,4% |

### Exemple IV-8: Emulsion bronzante sans soleil

| | |
|---|---|
| LANOL^{™} 99 : | 15% |
| Montanov^{™}68 : | 3,0% |
| PARSOL^{™} MCX : | 3,0% |
| Eau : | q.s.p. 100% |
| Dihydroxyacétone : | 5,0% |
| Phosphate monosodique : | 0,2% |
| Latex inverse auto-inversible (LI₅) : | 2,5% |
| Parfum : | 0,3% |
| SEPICIDE^{™} HB : | 0,8% |
| Hydroxyde de sodium : | q.s. pH=5. |

### Exemple IV-9: Crème de soin

| | |
|---|---|
| Cyclométhicone : | 10% |
| Latex inverse auto-inversible (LI₄) : | 2,8% |
| Montanov^{™}202 : | 4,5% |
| Conservateur : | 0,65% |
| Lysine : | 0,025% |
| EDTA (sel disodique) : | 0,05% |
| Gomme de xanthane : | 0,2% |
| Glycérine : | 3% |
| Eau : | qsp. 100% |

### Exemple IV-10: Crème solaire

| | |
|---|---|
| SIMULSOL^{™} 165 : | 3% |
| Montanov^{™}68 : | 2% |
| Benzoate C12-C15 : | 8% |
| PECOSIL^{™} PS 100 : | 2% |
| Diméthicone : | 2% |
| Cyclométhicone : | 5% |
| Para-méthoxy cinnamate d'octyle : | 6% |
| Benzophénone-3 : | 4% |
| Oxyde de Titane : | 8% |
| Gomme xanthane : | 0,2% |
| Butylèneglycol : | 5% |
| Eau déminéralisée : | qsp 100% |
| Latex inverse auto-inversible (LI₂) : | 1,5% |
| Conservateur, parfum : | qs |

### Exemple IV-11: Gel solaire et autobronzant

| | |
|---|---|
| Montanov^{™}68 : | 3,0% |
| Triheptanoate de glycéryle : | 10,0% |
| DEEPALINE^{™} PVB : | 1,05% |
| Latex inverse auto-inversible (LI₂): | 2,2% |
| Eau : | qs 100% |
| Dihydroxyacétone : | 5% |
| Parfum : | 0,1% |
| SEPICIDE^{™} HB : | 0,3% |
| 30 SEPICIDE^{™} CI : | 0,1% |
| PARSOL^{™} MCX : | 4,0% |

Les définitions des produits utilisés dans les exemples, sont les suivantes :
MICROPEARL^{™} M 100 est une poudre ultra fine au toucher très doux et à action matifiante commercialisée par la société MATSUMO
SEPICIDE^{™} CI, imidazolidine urée, est un agent conservateur commercialisé par la société SEPPIC.
SIMULSOL^{™} 165 est du stéarate de glycérol auto-émulsionnable commercialisée par la société SEPPIC.
SEPICIDE^{™} HB, qui est un mélange de phénoxyéthanol, de méthylparaben, d'éthylparaben, de propylparaben et de butylparaben, est un agent conservateur commercialisé par la société SEPPIC.
PARSOL^{™} MCX est du para-méthoxy cinnamate d'octyle ; commercialisé par la société GIVAUDAN.
LANOL^{™} 37T est du triheptanoate de glycérol, commercialisé par la société SEPPIC.
SOLAGUM^{™} L est un carraghénane commercialisé par la société SEPPIC.
EUSOLEX^{™} 4360 est un filtre solaire commercialisé par la société MERCK.
DEEPALINE^{™} PVB, est un hydrolysât de protéines de blé acylé commercialisé par la société SEPPIC.
PRIMOL^{™} 352 est une huile minérale commercialisée par la société EXXON.
PECOSIL^{™}PS 100 est du Dimethicone PEG-7 commercialisé par la société PHOENIX.
Montanov^{™}68 (nom INCI Cetearyl Alcohol (and) Cetearyl Glucoside) est un agent émulsionnant commercialisé par la Société SEPPIC
Montanov^{™}L (nom INCI C14-22 Alcohols (and) C12-20 Alkyl Glucoside) est un agent émulsionnant commercialisé par la Société SEPPIC.
Montanov^{™}202 (nom INCI Arachidyl Alcohol & Behenyl Alcohol & Arachidyl) ) est un agent émulsionnant commercialisé par la Société SEPPIC.

## Revendications

1. Utilisation d'un latex inverse auto-inversible comme agent épaississant et/ou émulsionnant et/ou stabilisant d'une composition topique cosmétique, ledit latex inverse auto-inversible étant un latex inverse auto-inversible d'un polyélectrolyte anionique réticulé (P) comprenant, pour 100% molaire :
**(a₁)** - d'une proportion supérieure ou égale à 30% molaire et inférieure ou égale à 100% molaire, d'unités monomériques issues de l'acide 2-méthyl 2-[(1-oxo 2-propènyl) amino] 1-propanesulfonique sous forme acide libre ou partiellement ou totalement salifiée ;
**(a₂)** - optionnellement d'une proportion supérieure à 0% molaire et inférieure ou égale à 70% molaire, d'unités monomériques issues d'au moins un monomère choisi parmi les éléments du groupe constitué par l'acide acrylique, l'acide méthacrylique, l'acide 2-carboxyéthyl acrylique, l'acide itaconique, l'acide maléique, l'acide 3-méthyl 3-[(1-oxo 2-propènyl) amino] butanoïque, la fonction carboxylique desdits monomères étant sous forme acide libre, partiellement salifiée ou totalement salifiée et/ou parmi les éléments du groupe constitué par l'acrylate de (2-hydroxy éthyle), l'acrylate de (2,3-dihydroxy propyle), le méthacrylate de (2-hydroxy éthyle), le méthacrylate de (2,3-dihydroxypropyle), ou le vinyl pyrrolidone ;
**(a₃)** - d'une proportion supérieure à 0% molaire et inférieure ou égale à 1% molaire d'unités monomériques issues d'au moins un monomère de réticulation (AR) diéthylénique ou polyéthylénique;
la somme des dites proportions molaires en unités monomériques selon a₁), a₂) et a₃) étant égale à 100% molaire;
ledit latex inverse auto-inversible étant une émulsion de type eau-dans-huile (E) comprenant pour 100% de sa masse :
**a)** - de 10% massique à 90% massique, dudit polyélectrolyte anionique réticulé (P) ;
**b) -** De 5% massique à 50% massique, d'une phase grasse constituée d'au moins une huile (H),
**c)** - De 1% massique à 50% massique d'eau
**d) -** De 0,5% massique à 10% massique d'un système émulsionnant de type eau-dans-huile (S₁), et
**e)** - De 2% massique à 10% massique d'un système émulsionnant de type huile-dans-eau (S₂) ;
la somme des proportions massiques en composés selon a), b), c), d) et e) étant égale à 100% massique ;
ledit latex inverse auto-inversible étant **caractérisée en ce que** ledit système émulsionnant de type huile-dans-eau (S₂) comprend pour 100% de sa masse :
**f)** - Une proportion supérieure ou égale à 50% massique et inférieure ou égale à 100% d'une composition (Ce) qui comprend pour 100% de sa masse :
**e₁)** - De 10% massique à 60% massique d'au moins un composé de formule (I) : HO-[CH₂-CH(OH)-CH₂-O]ₙ-H (I)
dans laquelle n représente un nombre entier supérieur ou égal à un et inférieur ou égal à quinze ;
**e₂)** - De 40% massique à 90 % massique d'au moins un composé de formule (II) :
R₁-(C=O)-[O-CH₂-CH(OH)-CH₂]ₚ-OH (II),
dans laquelle p, différent ou identique à n, représente un nombre entier supérieur ou égal à un et inférieur ou égal à quinze ; et dans laquelle le groupe R₁-(C=O)- représente un radical aliphatique saturé ou insaturé, linéaire ou ramifié, comportant de six à vingt-deux atomes de carbone, et optionnellement
**e₃)** - Jusqu'à 30% massique d'au moins une composition (C ₁₁) représentée par la formule (III) :
HO-[CH₂-CHOH-CH₂-O-]_{q}-(G)ᵣ-H (III),
dans laquelle q différent ou identique à n, représente un nombre entier supérieur ou égal à un et inférieur ou égal à 3, G représente le reste d'un sucre réducteur et r représente un nombre décimal supérieur ou égal à 1,05 et inférieur ou égal à 5,00,
ladite composition (C₁₁) consistant en un mélange des composés de formules (III₁), (III₂), (III₃), (III₄) et (III₅) :
HO-[CH₂-CHOH-CH₂-O-]_{q}-O-(G)₁-H (III₁),
HO-[CH₂-CHOH-CH₂-O-]_{q}-O-(G)₂-H (III₂),
HO-[CH₂-CHOH-CH₂-O-]_{q}-O-(G)₃-H (III₃),
HO-[CH₂-CHOH-CH₂-O-]_{q}-O-(G)₄-H (III₄),
HO-[CH₂-CHOH-CH₂-O-]_{q}-O-(G)₅-H (III₅),
en des proportions molaires en dits composés de formules (III₁), (III₂), (III₃), (III₄) et (III₅) respectivement égales à a₁, a₂, a₃, a₄ et a₅, telles que la somme (a₁+ a₂ + a₃ + a₄ + a₅) est égale à un, et que la somme (a₁ + 2a₂ + 3a₃ + 4a₄ + 5a₅) est égale à r ;
la somme des proportions massiques en composés selon e₁), e₂) et e₃) étant égale à 100% massique.

2. Utilisation telle que définie à la revendication 1, **caractérisé en ce que** ledit polyélectrolyte anionique réticulé (P) est un homopolymère de l'acide 2-méthyl 2-[(1-oxo 2-propènyl) amino] 1-propanesulfonique partiellement ou totalement salifié sous forme de sel de sodium ou de sel d'ammonium, réticulé par le triallylamine et/ou le méthylène-bis(acrylamide) ; un copolymère de l'acide 2-méthyl 2-[(1-oxo 2-propènyl) amino] 1-propanesulfonique et de l'acide acrylique partiellement ou totalement salifiés sous forme de sel de sodium ou de sel d'ammonium, réticulé par le triallylamine et/ou le méthylène-bis(acrylamide) ; un copolymère de l'acide 2-méthyl 2-[(1-oxo 2-propènyl) amino] 1-propanesulfonique (γ) partiellement ou totalement salifié sous forme de sel de sodium, et d'acide acrylique (δ) partiellement ou totalement salifié sous forme de sel de sodium dans un rapport molaire (γ)/(δ) supérieur ou égal à 30/70 et inférieur ou égal à 90/10 ; réticulé par le triallylamine et/ou le méthylène-bis(acrylamide) ; ou un copolymère de l'acide 2-méthyl 2-[(1-oxo 2-propènyl) amino] 1-propanesulfonique (γ) partiellement ou totalement salifié sous forme de sel de sodium, et de l'acide acrylique (δ)partiellement ou totalement salifié sous forme de sel de sodium dans un rapport molaire (γ)/(δ)supérieur ou égal à 40/60 et inférieur ou égal à 90/10, réticulé par le triallylamine et/ou le méthylène-bis(acrylamide).

3. Utilisation telle que définie à l'une quelconque des revendications 1 ou 2, **caractérisé en ce que** dans la formule (I) telle que définie précédemment, n représente un nombre entier supérieur ou égal à un et inférieur ou égal à dix, et **en ce que** dans la formule (II) telle que définie précédemment, p, identique ou différent de n, représente un nombre entier supérieur ou égal à un et inférieur ou égal à dix, et le groupe R₁-(C=O)-est choisi parmi les radicaux octanoyle, décanoyle, w-undécylènoyle, dodécanoyle, tétradécanoyle, hexadécanoyle, octadécanoyle, 9-octadécènoyle ou 9,12-octadécadiènoyle.

4. Utilisation telle que définie à l'une quelconque des revendications 1 à 3, **caractérisé en ce que** dans ledit système émulsionnant de type huile-dans-eau (S₂) ladite composition (Cₑ) telle que définie précédemment, consiste en, pour 100% de sa masse :
**e₁)** - De 10% massique à 60% massique d'au moins un composé de formule (I) telle que définie précédemment et
**e₂)** - De 40% massique à 90 % massique d'au moins un composé de formule (II) telle que définie précédemment.

5. Utilisation telle que définie à l'une quelconque des revendications 1 à 3, **caractérisé en ce que** dans la formule (III) telle que définie précédemment, q est égal à un, G représente le reste du glucose et r représente un nombre décimal supérieur ou égal à 1,05 et inférieur ou égal à 2,5.

6. Utilisation telle que définie à l'une quelconque des revendications 1 à 5, **caractérisé en ce que** ledit système émulsionnant de type huile-dans-eau (S₂) est ladite composition (Cₑ) telle que définie précédemment.

7. Composition cosmétique topique (F), **caractérisée en ce qu'**elle comprend comme agent épaississant, pour 100% de sa masse totale entre 0,1% et 10% massique d'un latex inverse auto-inversible d'un polyélectrolyte anionique réticulé (P) comprenant, pour 100% molaire :
**(a₁)** - d'une proportion supérieure ou égale à 30% molaire et inférieure ou égale à 100% molaire, d'unités monomériques issues de l'acide 2-méthyl 2-[(1-oxo 2-propènyl) amino] 1-propanesulfonique sous forme acide libre ou partiellement ou totalement salifiée ;
**(a₂)** - optionnellement d'une proportion supérieure à 0% molaire et inférieure ou égale à 70% molaire, d'unités monomériques issues d'au moins un monomère choisi parmi les éléments du groupe constitué par l'acide acrylique, l'acide méthacrylique, l'acide carboxyéthyl acrylique, l'acide itaconique, l'acide maléique, l'acide 3-méthyl 3-[(1-oxo 2-propènyl) amino] butanoïque, la fonction carboxylique desdits monomères étant sous forme acide libre, partiellement salifiée ou totalement salifiée et/ou parmi les éléments du groupe constitué par l'acrylate de (2-hydroxy éthyle), l'acrylate de (2,3-dihydroxy propyle), le méthacrylate de (2-hydroxy éthyle), le méthacrylate de (2,3-dihydroxypropyle), ou le vinyl pyrrolidone ;
**(a₃)** - d'une proportion supérieure à 0% molaire et inférieure ou égale à 1% molaire d'unités monomériques issues d'au moins un monomère de réticulation (AR) diéthylénique ou polyéthylénique;
la somme des dites proportions molaires en unités monomériques selon a₁), a₂) et a₃) étant égale à 100% molaire;
ledit latex inverse auto-inversible étant une émulsion de type eau-dans-huile (E) comprenant pour 100% de sa masse :
**a)** - de 10% massique à 90% massique, dudit polyélectrolyte anionique réticulé (P) ;
**b)** - De 5% massique à 50% massique, d'une phase grasse constituée d'au moins une huile (H),
**c)** - De 1% massique à 50% massique d'eau,
**d)** - De 0,5% massique à 10% massique d'un système émulsionnant de type eau-dans-huile (S₁), et
**e)** - De 2% massique à 10% massique d'un système émulsionnant de type huile-dans-eau (S₂) ;
la somme des proportions massiques en composés selon a), b), c), d) et e) étant égale à 100% massique ;
ledit latex inverse auto-inversible étant **caractérisée en ce que** ledit système émulsionnant de type huile-dans-eau (S₂) comprend pour 100% de sa masse :
**f)** - Une proportion supérieure ou égale à 50% massique et inférieure ou égale à 100% d'une composition (Ce) qui comprend pour 100% de sa masse :
**e₁)** - De 10% massique à 60% massique d'au moins un composé de formule (I) :
HO-[CH₂-CH(OH)-CH₂-O]ₙ-H (I)
dans laquelle n représente un nombre entier supérieur ou égal à un et inférieur ou égal à quinze ; **e₂)** - De 40% massique à 90 % massique d'au moins un composé de formule (II) :
R₁-(C=O)-[O-CH₂-CH(OH)-CH₂]ₚ-OH (II),
dans laquelle p, différent ou identique à n, représente un nombre entier supérieur ou égal à un et inférieur ou égal à quinze ; et dans laquelle le groupe R₁-(C=O)- représente un radical aliphatique saturé ou insaturé, linéaire ou ramifié, comportant de six à vingt-deux atomes de carbone, et optionnellement **e₃)** - Jusqu'à 30% massique d'au moins une composition (C ₁₁) représentée par la formule (III) :
HO-[CH₂-CHOH-CH₂-O-]_{q}-(G)ᵣ-H (III),
dans laquelle q différent ou identique à n, représente un nombre entier supérieur ou égal à un et inférieur ou égal à 3, G représente le reste d'un sucre réducteur et r représente un nombre décimal supérieur ou égal à 1,05 et inférieur ou égal à 5,00,
ladite composition (C₁₁) consistant en un mélange des composés de formules (III₁), (III₂), (III₃), (III₄) et (III₅) :
HO-[CH₂-CHOH-CH₂-O-]_{q}-O-(G)₁-H (III₁),
HO-[CH₂-CHOH-CH₂-O-]_{q}-O-(G)₂-H (III₂),
HO-[CH₂-CHOH-CH₂-O-]_{q}-O-(G)₃-H (III₃),
HO-[CH₂-CHOH-CH₂-O-]_{q}-O-(G)₄-H (III₄),
HO-[CH₂-CHOH-CH₂-O-]_{q}-O-(G)₅-H (III₅),
en des proportions molaires en dits composés de formules (III₁), (III₂), (III₃), (III₄) et (III₅) respectivement égales à a₁, a₂, a₃, a₄ et a₅, telles que la somme (a₁+ a₂ + a₃ + a₄ + a₅) est égale à un, et que la somme (a₁ + 2a₂ + 3a₃ + 4a₄ + 5a₅) est égale à r ;
la somme des proportions massiques en composés selon e₁), e₂) et e₃) étant égale à 100% massique.

8. Composition pharmaceutique topique (G) **caractérisée en ce qu'**elle comprend comme agent épaississant, pour 100% de sa masse totale entre 0,1% et 10% massique d'un latex inverse auto-inversible d'un polyélectrolyte anionique réticulé (P) comprenant, pour 100% molaire :
**(a₁)** - d'une proportion supérieure ou égale à 30% molaire et inférieure ou égale à 100% molaire, d'unités monomériques issues de l'acide 2-méthyl 2-[(1-oxo 2-propènyl) amino] 1-propanesulfonique sous forme acide libre ou partiellement ou totalement salifiée ;
**(a₂)** - optionnellement d'une proportion supérieure à 0% molaire et inférieure ou égale à 70% molaire, d'unités monomériques issues d'au moins un monomère choisi parmi les éléments du groupe constitué par l'acide acrylique, l'acide méthacrylique, l'acide 2-carboxyéthyl acrylique, l'acide itaconique, l'acide maléique, l'acide 3-méthyl 3-[(1-oxo 2-propènyl) amino] butanoïque, la fonction carboxylique desdits monomères étant sous forme acide libre, partiellement salifiée ou totalement salifiée et/ou parmi les éléments du groupe constitué par l'acrylate de (2-hydroxy éthyle), l'acrylate de (2,3-dihydroxy propyle), le méthacrylate de (2-hydroxy éthyle), le méthacrylate de (2,3-dihydroxypropyle), ou le vinyl pyrrolidone ;
**(a₃)** - d'une proportion supérieure à 0% molaire et inférieure ou égale à 1% molaire d'unités monomériques issues d'au moins un monomère de réticulation (AR) diéthylénique ou polyéthylénique;
la somme des dites proportions molaires en unités monomériques selon a₁), a₂) et a₃) étant égale à 100% molaire;
ledit latex inverse auto-inversible étant une émulsion de type eau-dans-huile (E) comprenant pour 100% de sa masse :
**a)** - de 10% massique à 90% massique, dudit polyélectrolyte anionique réticulé (P) ;
**b) -** De 5% massique à 50% massique, d'une phase grasse constituée d'au moins une huile (H),
**c)** - De 1% massique à 50% massique d'eau,
**d) -** De 0,5% massique à 10% massique d'un système émulsionnant de type eau-dans-huile (S₁), et
**e)** - De 2% massique à 10% massique d'un système émulsionnant de type huile-dans-eau (S₂) ;
la somme des proportions massiques en composés selon a), b), c), d) et e) étant égale à 100% massique ;
ledit latex inverse auto-inversible étant **caractérisée en ce que** ledit système émulsionnant de type huile-dans-eau (S₂) comprend pour 100% de sa masse :
**f)** - Une proportion supérieure ou égale à 50% massique et inférieure ou égale à 100% d'une composition (Ce) qui comprend pour 100% de sa masse :
**e₁)** - De 10% massique à 60% massique d'au moins un composé de formule (I) :
HO-[CH₂-CH(OH)-CH₂-O]ₙ-H (I)
dans laquelle n représente un nombre entier supérieur ou égal à un et inférieur ou égal à quinze ;
**e₂)** - De 40% massique à 90 % massique d'au moins un composé de formule (II) :
R₁-(C=O)-[O-CH₂-CH(OH)-CH₂]ₚ-OH (II),
dans laquelle p, différent ou identique à n, représente un nombre entier supérieur ou égal à un et inférieur ou égal à quinze ; et dans laquelle le groupe R₁-(C=O)- représente un radical aliphatique saturé ou insaturé, linéaire ou ramifié, comportant de six à vingt-deux atomes de carbone, et optionnellement
**e₃)** - Jusqu'à 30% massique d'au moins une composition (C ₁₁) représentée par la formule (III) :
HO-[CH₂-CHOH-CH₂-O-]_{q}-(G)ᵣH (III),
dans laquelle q différent ou identique à n, représente un nombre entier supérieur ou égal à un et inférieur ou égal à 3, G représente le reste d'un sucre réducteur et r représente un nombre décimal supérieur ou égal à 1,05 et inférieur ou égal à 5,00,
ladite composition (C₁₁) consistant en un mélange des composés de formules (III₁), (III₂), (III₃), (III₄) et (III₅) :
HO-[CH₂-CHOH-CH₂-O-]_{q}-O-(G)₁-H (III₁),
HO-[CH₂-CHOH-CH₂-O-]_{q}-O-(G)₂-H (III₂),
HO-[CH₂-CHOH-CH₂-O-]_{q}-O-(G)₃-H (III₃),
HO-[CH₂-CHOH-CH₂-O-]_{q}-O-(G)₄-H (III₄),
HO-[CH₂-CHOH-CH₂-O-]_{q}-O-(G)₅-H (III₅),
en des proportions molaires en dits composés de formules (III₁), (III₂), (III₃), (III₄) et (III₅) respectivement égales à a₁, a₂, a₃, a₄ et a₅, telles que la somme (a₁+ a₂ + a₃ + a₄ + a₅) est égale à un, et que la somme (a₁ + 2a₂ + 3a₃ + 4a₄ + 5a₅) est égale à r ;
la somme des proportions massiques en composés selon e₁), e₂) et e₃) étant égale à 100% massique..

9. Utilisation d'une composition (C ₑ) comme agent inverseur d'un latex inverse d'un polyélectrolyte anionique réticulé (P) comprenant pour 100% molaire :
**(a₁)** - d'une proportion supérieure ou égale à 30% molaire et inférieure ou égale à 100% molaire, d'unités monomériques issues de l'acide 2-méthyl 2-[(1-oxo 2-propènyl) amino] 1-propanesulfonique sous forme acide libre ou partiellement ou totalement salifiée ; **(a₂)** - optionnellement d'une proportion supérieure à 0% molaire et inférieure ou égale à 70% molaire, d'unités monomériques issues d'au moins un monomère choisi parmi les éléments du groupe constitué par l'acide acrylique, l'acide méthacrylique, l'acide 2-carboxyéthyl acrylique, l'acide itaconique, l'acide maléique, l'acide 3-méthyl 3-[(1-oxo 2-propènyl) amino] butanoïque, la fonction carboxylique desdits monomères étant sous forme acide libre, partiellement salifiée ou totalement salifiée et/ou parmi les éléments du groupe constitué par l'acrylate de (2-hydroxy éthyle), l'acrylate de (2,3-dihydroxy propyle), le méthacrylate de (2-hydroxy éthyle), le méthacrylate de (2,3-dihydroxypropyle), ou le vinyl pyrrolidone ;
**(a₃)** - d'une proportion supérieure à 0% molaire et inférieure ou égale à 1% molaire d'unités monomériques issues d'au moins un monomère de réticulation (AR) diéthylénique ou polyéthylénique;
la somme des dites proportions molaires en unités monomériques selon a₁), a₂) et a₃) étant égale à 100% molaire;
ledit latex inverse étant une émulsion de type eau-dans-huile (E) comprenant pour 100% de sa masse :
**a)** - de 10% massique à 90% massique, dudit polyélectrolyte anionique réticulé (P) ;
**b) -** De 5% massique à 50% massique, d'une phase grasse constituée d'au moins une huile (H),
**c)** - De 1% massique à 50% massique d'eau, et
**d) -** De 0,5% massique à 10% massique d'un système émulsionnant de type eau-dans-huile (S₁).
la somme des proportions massiques en composés selon a), b), c) et d) étant égale à 100% massique,
ladite composition (Cₑ) comprenant pour 100% de sa masse :
**e₁)** - De 10% massique à 60% massique d'au moins un composé de formule (I) :
HO-[CH₂-CH(OH)-CH₂-O]ₙ-H (I)
dans laquelle n représente un nombre entier supérieur ou égal à un et inférieur ou égal à quinze ;
**e₂)** - De 40% massique à 90 % massique d'au moins un composé de formule (II) :
R₁-(C=O)-[O-CH₂-CH(OH)-CH₂]ₚ-OH (II),
dans laquelle p, différent ou identique à n, représente un nombre entier supérieur ou égal à un et inférieur ou égal à quinze et le groupe R₁-(C=O)- représente un radical aliphatique saturé ou insaturé, linéaire ou ramifié, comportant de six à vingt-deux atomes de carbone, et optionnellement
**e₃)** - Jusqu'à 30% massique d'au moins une composition (C ₁₁) représentée par la formule (III) :
HO-[CH₂-CHOH-CH₂-O-]_{q}-(G)ᵣ-H (III),
formule (III) dans laquelle q, différent ou identique à n, représente un nombre entier supérieur ou égal à un et inférieur ou égal à 3, G représente le reste d'un sucre réducteur et r représente un nombre décimal supérieur ou égal à 1,05 et inférieur ou égal à 5,00, ladite composition (C₁₁) consistant en un mélange des composés de formules (III₁), (III₂), (III₃), (III₄) et (III₅) :
HO-[CH₂-CHOH-CH₂-O-]_{q}-O-(G)₁-H (III₁),
HO-[CH₂-CHOH-CH₂-O-]_{q}-O-(G)₂-H (III₂),
HO-[CH₂-CHOH-CH₂-O-]_{q}-O-(G)₃-H (III₃),
HO-[CH₂-CHOH-CH₂-O-]_{q}-O-(G)₄-H (III₄),
HO-[CH₂-CHOH-CH₂-O-]_{q}-O-(G)₅-H (III₅),
en des proportions molaires en dits composés de formules (III₁), (III₂), (III₃), (III₄) et (III₅) respectivement égales à a₁, a₂, a₃, a₄ et a₅, telles que la somme a₁+ a₂ + a₃ + a₄ + a₅ est égale à un, et que la somme a₁ + 2a₂ + 3a₃ + 4a₄ + 5a₅ est égale à r ;
la somme des proportions massiques en composés selon e₁), e₂) et e₃) étant égale à 100% massique.

10. Utilisation telle que définie à la revendication 9, **caractérisée en ce que** dans la formule (I) telle que définie précédemment, n représente un nombre entier supérieur ou égal à un et inférieur ou égal à dix, **et en ce que** dans la formule (II) telle que définie précédemment, p, identique ou différent de n, représente un nombre entier supérieur ou égal à un et inférieur ou égal à dix, et le groupe R₁-(C=O)- est choisi parmi les radicaux octanoyle, décanoyle, w-undécylènoyle, dodécanoyle, tétradécanoyle, hexadécanoyle, octadécanoyle, 9-octadécènoyle ou 9,12-octadécadiènoyle.

11. Utilisation telle que définie à l'une ou quelconque des revendications 9 ou 10, **caractérisée en ce qu'**elle consiste en, pour 100% de sa masse :
**e₁)** - De 10% massique à 60% massique d'au moins un composé de formule (I) telle que définie précédemment et
**e₂)** - De 40% massique à 90 % massique d'au moins un composé de formule (II) telle que définie précédemment.

12. Utilisation telle que définie à l'une ou quelconque des revendications 9 ou 10, **caractérisée en ce que** dans la formule (III) telle que définie précédemment, q est égal à un, G représente le reste du glucose et r représente un nombre décimal supérieur ou égal à 1,05 et inférieur ou égal à 2,5.

## Patentansprüche

1. Verwendung eines selbstinvertierbaren inversen Latex als Verdickungsmittel und/oder Emulgator und/oder Stabilisator für eine topische kosmetische Zusammensetzung, wobei es sich bei dem selbstinvertierbaren inversen Latex um einen selbstinvertierbaren inversen Latex eines vernetzten anionischen Polyelektrolyten (P) handelt, der pro 100 Mol-% Folgendes umfasst:
(a₁) - einen Anteil von mehr als oder gleich 30 Mol-% und weniger als oder gleich 100 Mol-% an Monomereinheiten, die von 2-Methyl-2-[(1-oxo-2-propenyl)amino]-1-propansulfonsäure, in Form der freien Säure oder partiell oder vollständig versalzt, stammen;
(a₂) - gegebenenfalls einen Anteil von mehr als 0 Mol-% und weniger als oder gleich 70 Mol-% an Monomereinheiten, die von mindestens einem Monomer stammen, das aus den Elementen der aus Acrylsäure, Methacrylsäure, 2-Carboxyethylacrylsäure, Itaconsäure, Maleinsäure, 3-Methyl-3-[(1-oxo-2-propenyl)amino]butansäure bestehenden Gruppe, wobei die Carboxylfunktion der Monomere in Form der freien Säure, partiell versalzt oder vollständig versalzt ist, und/oder aus den Elementen der aus (2- Hydroxyethyl)acrylat, (2,3-Dihydroxypropyl)acrylat, (2-Hydroxyethyl)methacrylat, (2,3-Dihydroxypropyl)methacrylat bestehenden Gruppe oder Vinylpyrrolidon ausgewählt ist;
(a₃) - einen Anteil von mehr als 0 Mol-% und weniger als oder gleich 1 Mol-% an Monomereinheiten, die von mindestens einem diethylenisch oder polyethylenisch vernetzenden Monomer (AR) stammen;
wobei die Summe der molaren Anteile der Monomereinheiten gemäß a₁), a₂) und a₃) gleich 100 Mol-% ist;
wobei es sich bei dem selbstinvertierbaren inversen Latex um eine Emulsion des Wasser-in-Öl-Typs (E) handelt, die pro 100 % ihrer Masse Folgendes umfasst:
a) - von 10 Massen-% bis 90 Massen-% des vernetzten anionischen Polyelektrolyten (P);
b) - von 5 Massen-% bis 50 Massen-% einer aus mindestens einem Öl (H) bestehenden Fettphase,
c) - von 1 Massen-% bis 50 Massen-% Wasser,
d) - von 0,5 Massen-% bis 10 Massen-% eines Emulgiersystems des Wasser-in-Öl-Typs (S₁) und
e) - von 2 Massen-% bis 10 Massen-% eines Emulgiersystems des Öl-in-Wasser-Typs (S₂);
wobei die Summe der Massenanteile der Verbindungen gemäß a), b), c), d) und e) gleich 100 Massen-% ist;
wobei der selbstinvertierbare inverse Latex **dadurch gekennzeichnet ist, dass** das Emulgiersystem des Öl-in-Wasser-Typs (S₂) pro 100 % seiner Masse Folgendes umfasst:
f) - einen Anteil von mehr als oder gleich 50 Massen-% und weniger als oder gleich 100 % einer Zusammensetzung (Ce), die pro 100 % ihrer Masse Folgendes umfasst:
e₁) - von 10 Massen-% bis 60 Massen-% mindestens einer Verbindung der Formel (I):
HO-[CH₂-CH(OH)-CH₂-O]ₙ-H (I)
worin n eine ganze Zahl größer als oder gleich eins und kleiner als oder gleich fünfzehn darstellt;
e₂) - von 40 Massen-% bis 90 Massen-% mindestens einer Verbindung der Formel (II):
R₁-(C=O)-[O-CH₂-CH(OH)-CH₂]ₚ-OH (II),
worin p, das verschieden von oder gleich n ist, eine ganze Zahl größer als oder gleich eins und kleiner als oder gleich fünfzehn darstellt und worin die Gruppe R₁-(C=O)- einen geraden oder verzweigten, gesättigten oder ungesättigten aliphatischen Rest mit sechs bis zweiundzwanzig Kohlenstoffatomen darstellt, und gegebenenfalls
e₃) - bis zu 30 Massen-% mindestens einer Zusammensetzung (C₁₁), dargestellt durch die Formel (III):
HO-[CH₂-CHOH-CH₂-O-]_{q}-(G)ᵣ-H (III),
worin q, das verschieden von oder gleich n ist, eine ganze Zahl größer als oder gleich eins und kleiner als oder gleich 3 darstellt, G den Rest eines reduzierenden Zuckers darstellt und r eine Dezimalzahl größer als oder gleich 1,05 und kleiner als oder gleich 5,00 darstellt,
wobei die Zusammensetzung (C₁₁) aus einem Gemisch der Verbindungen der Formeln (III₁), (III₂), (III₃), (III₄) und (III₅):
HO-[CH₂-CHOH-CH₂-O-]_{q}-O-(G)₁-H (III₁),
HO-[CH₂-CHOH-CH₂-O-]_{q}-O-(G)₂-H (III₂),
HO-[CH₂-CHOH-CH₂-O-]_{q}-O-(G)₃-H (III₃),
HO-[CH₂-CHOH-CH₂-O-]_{q}-O-(G)₄-H (III₄),
HO-[CH₂-CHOH-CH₂-O-]_{q}-O-(G)₅-H (III₅),
in molaren Anteilen dieser Verbindungen der Formeln (III₁), (III₂), (III₃), (III₄) und (III₅) besteht, die gleich a₁, a₂, a₃, a₄ bzw. a₅ sind, so dass die Summe (a₁+ a₂ + a₃ + a₄ + a₅) gleich eins ist und die Summe (a₁ + 2a₂ + 3a₃ + 4a₄ + 5a₅) gleich r ist;
wobei die Summe der Massenanteile der Verbindungen gemäß e_{d}, e₂) und e₃) gleich 100 Massen-% ist.

2. Verwendung nach Anspruch 1, **dadurch gekennzeichnet, dass** es sich bei dem vernetzten anionischen Polyelektrolyt (P) um Folgendes handelt: ein durch Triallylamin und/oder Methylenbis(acrylamid) vernetztes Homopolymer von partiell oder vollständig in Form des Natriumsalzes oder Ammoniumsalzes versalzter 2-Methyl-2-[(1-oxo-2-propenyl)amino]-1-propansulfonsäure; ein durch Triallylamin und/oder Methylenbis(acrylamid) vernetztes Copolymer von partiell oder vollständig in Form des Natriumsalzes oder Ammoniumsalzes versalzter 2-Methyl-2-[(1-oxo-2-propenyl)amino]-1-propansulfonsäure und Acrylsäure; ein durch Triallylamin und/oder Methylenbis(acrylamid) vernetztes Copolymer von partiell oder vollständig in Form des Natriumsalzes versalzter 2-Methyl-2-[(1-oxo-2-propenyl)amino]-1-propansulfonsäure (γ) und partiell oder vollständig in Form des Natriumsalzes versalzter Acrylsäure (δ) in einem molaren Verhältnis (γ)/(δ) größer als oder gleich 30/70 und kleiner als oder gleich 90/10 oder ein durch Triallylamin und/oder Methylenbis(acrylamid) vernetztes Copolymer von partiell oder vollständig in Form des Natriumsalzes versalzter 2-Methyl-2-[(1-oxo-2-propenyl)amino]-1-propansulfonsäure (γ) und partiell oder vollständig in Form des Natriumsalzes versalzter Acrylsäure (δ) in einem molaren Verhältnis (γ)/(δ) größer als oder gleich 40/60 und kleiner als oder gleich 90/10.

3. Verwendung nach Anspruch 1 oder 2, **dadurch gekennzeichnet, dass** in der Formel (I) gemäß obiger Definition n eine ganze Zahl größer als oder gleich eins und kleiner als oder gleich zehn darstellt, und dadurch, dass in der Formel (II) gemäß obiger Definition p, das gleich oder verschieden von n ist, eine ganze Zahl größer als oder gleich eins und kleiner als oder gleich zehn darstellt und die Gruppe R₁-(C=O)- aus Octanoyl-, Decanoyl-, w-Undecylenoyl-, Dodecanoyl-, Tetradecanoyl-, Hexadecanoyl-, Octadecanoyl-, 9-Octadecenoyl- oder 9,12-Octadecadienoyl-Resten ausgewählt ist.

4. Verwendung nach einem der Ansprüche 1 bis 3, **dadurch gekennzeichnet, dass** in dem Emulgiersystem des Öl-in-Wasser-Typs (S₂) die Zusammensetzung (Cₑ) gemäß obiger Definition pro 100 % ihrer Masse aus Folgendem besteht:
e₁) - von 10 Massen-% bis 60 Massen-% mindestens einer Verbindung der Formel (I) gemäß obiger Definition und
e₂) - von 40 Massen-% bis 90 Massen-% von mindestens einer Verbindung der Formel (II) gemäß obiger Definition.

5. Verwendung nach einem der Ansprüche 1 bis 3, **dadurch gekennzeichnet, dass** in der Formel (III) gemäß obiger Definition q gleich eins ist, G den Rest von Glucose darstellt und r eine Dezimalzahl größer als oder gleich 1,05 und kleiner als oder gleich 2,5 darstellt.

6. Verwendung nach einem der Ansprüche 1 bis 5, **dadurch gekennzeichnet, dass** es sich bei dem Emulgiersystem des Öl-in-Wasser-Typs (S₂) um die Zusammensetzung (Cₑ) gemäß obiger Definition handelt.

7. Topische kosmetische Zusammensetzung (F), **dadurch gekennzeichnet, dass** sie als Verdickungsmittel pro 100 % ihrer Gesamtmasse zwischen 0,1 Massen-% und 10 Massen-% eines selbstinvertierbaren inversen Latex eines vernetzten anionischen Polyelektrolyten (P) umfasst, der pro 100 Mol-% Folgendes umfasst:
(a₁) - einen Anteil von mehr als oder gleich 30 Mol-% und weniger als oder gleich 100 Mol-% an Monomereinheiten, die von 2-Methyl-2-[(1-oxo-2-propenyl)amino]-1-propansulfonsäure, in Form der freien Säure oder partiell oder vollständig versalzt, stammen;
(a₂) - gegebenenfalls einen Anteil von mehr als 0 Mol-% und weniger als oder gleich 70 Mol-% an Monomereinheiten, die von mindestens einem Monomer stammen, das aus den Elementen der aus Acrylsäure, Methacrylsäure, 2-Carboxyethylacrylsäure, Itaconsäure, Maleinsäure, 3-Methyl-3-[(1-oxo-2-propenyl)amino]butansäure bestehenden Gruppe, wobei die Carboxylfunktion der Monomere in Form der freien Säure, partiell versalzt oder vollständig versalzt ist, und/oder aus den Elementen der aus (2- Hydroxyethyl)acrylat, (2,3-Dihydroxypropyl)acrylat, (2-Hydroxyethyl)methacrylat, (2,3-Dihydroxypropyl)methacrylat bestehenden Gruppe oder Vinylpyrrolidon ausgewählt ist;
(a₃) - einen Anteil von mehr als 0 Mol-% und weniger als oder gleich 1 Mol-% an Monomereinheiten, die von mindestens einem diethylenisch oder polyethylenisch vernetzenden Monomer (AR) stammen;
wobei die Summe der molaren Anteile der Monomereinheiten gemäß a₁) , a₂) und a₃) gleich 100 Mol-% ist;
wobei es sich bei dem selbstinvertierbaren inversen Latex um eine Emulsion des Wasser-in-Öl-Typs (E) handelt, die pro 100 % ihrer Masse Folgendes umfasst:
a) - von 10 Massen-% bis 90 Massen-% des vernetzten anionischen Polyelektrolyten (P);
b) - von 5 Massen-% bis 50 Massen-% einer aus mindestens einem Öl (H) bestehenden Fettphase,
c) - von 1 Massen-% bis 50 Massen-% Wasser,
d) - von 0,5 Massen-% bis 10 Massen-% eines Emulgiersystems des Wasser-in-Öl-Typs (S₁) und
e) - von 2 Massen-% bis 10 Massen-% eines Emulgiersystems des Öl-in-Wasser-Typs (S₂);
wobei die Summe der Massenanteile der Verbindungen gemäß a), b), c), d) und e) gleich 100 Massen-% ist;
wobei der selbstinvertierbare inverse Latex **dadurch gekennzeichnet ist, dass** das Emulgiersystem des Öl-in-Wasser-Typs (S₂) pro 100 % seiner Masse Folgendes umfasst:
f) - einen Anteil von mehr als oder gleich 50 Massen-% und weniger als oder gleich 100 % einer Zusammensetzung (Ce), die pro 100 % ihrer Masse Folgendes umfasst:
e₁) - von 10 Massen-% bis 60 Massen-% mindestens einer Verbindung der Formel (I):
HO-[CH₂-CH(OH)-CH₂-O]ₙ-H (I)
worin n eine ganze Zahl größer als oder gleich eins und kleiner als oder gleich fünfzehn darstellt;
e₂) - von 40 Massen-% bis 90 Massen-% mindestens einer Verbindung der Formel (II):
R₁-(C=O)-[O-CH₂-CH(OH)-CH₂]ₚ-OH (II),
worin p, das verschieden von oder gleich n ist, eine ganze Zahl größer als oder gleich eins und kleiner als oder gleich fünfzehn darstellt und worin die Gruppe R₁-(C=O)- einen geraden oder verzweigten, gesättigten oder ungesättigten aliphatischen Rest mit sechs bis zweiundzwanzig Kohlenstoffatomen darstellt, und gegebenenfalls
e₃) - bis zu 30 Massen-% mindestens einer Zusammensetzung (C₁₁), dargestellt durch die Formel (III):
HO-[CH₂-CHOH-CH₂-O-]_{q}-(G)ᵣ-H (III),
worin q, das verschieden von oder gleich n ist, eine ganze Zahl größer als oder gleich eins und kleiner als oder gleich 3 darstellt, G den Rest eines reduzierenden Zuckers darstellt und r eine Dezimalzahl größer als oder gleich 1,05 und kleiner als oder gleich 5,00 darstellt,
wobei die Zusammensetzung (C₁₁) aus einem Gemisch der Verbindungen der Formeln (III₁), (III₂), (III₃), (III₄) und (III₅):
HO-[CH₂-CHOH-CH₂-O-]_{q}-O-(G)₁-H (III₁),
HO-[CH₂-CHOH-CH₂-O-]_{q}-O-(G)₂-H (III₂),
HO-[CH₂-CHOH-CH₂-O-]_{q}-O-(G)₃-H (III₃),
HO-[CH₂-CHOH-CH₂-O-]_{q}-O-(G)₄-H (III₄),
HO-[CH₂-CHOH-CH₂-O-]_{q}-O-(G)₅-H (III₅),
in molaren Anteilen dieser Verbindungen der Formeln (III₁), (III₂), (III₃), (III₄) und (III₅) besteht, die gleich a₁, a₂, a₃, a₄ bzw. a₅ sind, so dass die Summe (a₁+ a₂ + a₃ + a₄ + a₅) gleich eins ist und die Summe (a₁ + 2a₂ + 3a₃ + 4a₄ + 5a₅) gleich r ist;
wobei die Summe der Massenanteile der Verbindungen gemäß e₁), e₂) und e₃) gleich 100 Massen-% ist.

8. Topische pharmazeutische Zusammensetzung (G), **dadurch gekennzeichnet, dass** sie als Verdickungsmittel pro 100 % ihrer Gesamtmasse zwischen 0,1 Massen-% und 10 Massen-% eines selbstinvertierbaren inversen Latex eines vernetzten anionischen Polyelektrolyten (P) umfasst, der pro 100 Mol-% Folgendes umfasst:
(a₁) - einen Anteil von mehr als oder gleich 30 Mol-% und weniger als oder gleich 100 Mol-% an Monomereinheiten, die von 2-Methyl-2-[(1-oxo-2-propenyl)amino]-1-propansulfonsäure, in Form der freien Säure oder partiell oder vollständig versalzt, stammen;
(a₂) - gegebenenfalls einen Anteil von mehr als 0 Mol-% und weniger als oder gleich 70 Mol-% an Monomereinheiten, die von mindestens einem Monomer stammen, das aus den Elementen der aus Acrylsäure, Methacrylsäure, 2-Carboxyethylacrylsäure, Itaconsäure, Maleinsäure, 3-Methyl-3-[(1-oxo-2-propenyl)amino]butansäure bestehenden Gruppe, wobei die Carboxylfunktion der Monomere in Form der freien Säure, partiell versalzt oder vollständig versalzt ist, und/oder aus den Elementen der aus (2- Hydroxyethyl)acrylat, (2,3-Dihydroxypropyl)acrylat, (2-Hydroxyethyl)methacrylat, (2,3-Dihydroxypropyl)methacrylat bestehenden Gruppe oder Vinylpyrrolidon ausgewählt ist;
(a₃) - einen Anteil von mehr als 0 Mol-% und weniger als oder gleich 1 Mol-% an Monomereinheiten, die von mindestens einem diethylenisch oder polyethylenisch vernetzenden Monomer (AR) stammen;
wobei die Summe der molaren Anteile der Monomereinheiten gemäß a₁), a₂) und a₃) gleich 100 Mol-% ist;
wobei es sich bei dem selbstinvertierbaren inversen Latex um eine Emulsion des Wasser-in-Öl-Typs (E) handelt, die pro 100 % ihrer Masse Folgendes umfasst:
a) - von 10 Massen-% bis 90 Massen-% des vernetzten anionischen Polyelektrolyten (P);
b) - von 5 Massen-% bis 50 Massen-% einer aus mindestens einem Öl (H) bestehenden Fettphase,
c) - von 1 Massen-% bis 50 Massen-% Wasser,
d) - von 0,5 Massen-% bis 10 Massen-% eines Emulgiersystems des Wasser-in-Öl-Typs (S₁) und
e) - von 2 Massen-% bis 10 Massen-% eines Emulgiersystems des Öl-in-Wasser-Typs (S₂);
wobei die Summe der Massenanteile der Verbindungen gemäß a), b), c), d) und e) gleich 100 Massen-% ist;
wobei der selbstinvertierbare inverse Latex **dadurch gekennzeichnet ist, dass** das Emulgiersystem des Öl-in-Wasser-Typs (S₂) pro 100 % seiner Masse Folgendes umfasst:
f) - einen Anteil von mehr als oder gleich 50 Massen-% und weniger als oder gleich 100 % einer Zusammensetzung (Ce), die pro 100 % ihrer Masse Folgendes umfasst:
e₁) - von 10 Massen-% bis 60 Massen-% mindestens einer Verbindung der Formel (I):
HO-[CH₂-CH(OH)-CH₂-O]ₙ-H (I)
worin n eine ganze Zahl größer als oder gleich eins und kleiner als oder gleich fünfzehn darstellt;
e₂) - von 40 Massen-% bis 90 Massen-% mindestens einer Verbindung der Formel (II):
R₁-(C=O)-[O-CH₂-CH(OH)-CH₂]ₚ-OH (II),
worin p, das verschieden von oder gleich n ist, eine ganze Zahl größer als oder gleich eins und kleiner als oder gleich fünfzehn darstellt und worin die Gruppe R₁-(C=O)- einen geraden oder verzweigten, gesättigten oder ungesättigten aliphatischen Rest mit sechs bis zweiundzwanzig Kohlenstoffatomen darstellt, und gegebenenfalls
e₃) - bis zu 30 Massen-% mindestens einer Zusammensetzung (C₁₁), dargestellt durch die Formel (III):
HO-[CH₂-CHOH-CH₂-O-]_{q}-(G)ᵣ-H (III),
worin q, das verschieden von oder gleich n ist, eine ganze Zahl größer als oder gleich eins und kleiner als oder gleich 3 darstellt, G den Rest eines reduzierenden Zuckers darstellt und r eine Dezimalzahl größer als oder gleich 1,05 und kleiner als oder gleich 5,00 darstellt,
wobei die Zusammensetzung (C₁₁) aus einem Gemisch der Verbindungen der Formeln (III₁), (III₂), (III₃), (III₄) und (III₅):
HO-[CH₂-CHOH-CH₂-O-]_{q} -O-(G)₁-H (III₁),
HO-[CH₂-CHOH-CH₂-O-]_{q} -O-(G)₂-H (III₂),
HO-[CH₂-CHOH-CH₂-O-]_{q} -O-(G)₃-H (III₃),
HO-[CH₂-CHOH-CH₂-O-]_{q} -O-(G)₄-H (III₄),
HO-[CH₂-CHOH-CH₂-O-]_{q} -O-(G)₅-H (III₃),
in molaren Anteilen dieser Verbindungen der Formeln (III₁), (III₂), (III₃), (III₄) und (III₅) besteht, die gleich a₁, a₂, a₃, a₄ bzw. a₅ sind, so dass die Summe (a₁+ a₂ + a₃ + a₄ + a₅) gleich eins ist und die Summe (a₁ + 2a₂ + 3a₃ + 4a₄ + 5as) gleich r ist;
wobei die Summe der Massenanteile der Verbindungen gemäß e₁), e₂) und e₃) gleich 100 Massen-% ist.

9. Verwendung einer Zusammensetzung (Cₑ) als Invertierungsmittel eines inversen Latex eines vernetzten anionischen Polyelektrolyten (P), der pro 100 Mol-% Folgendes umfasst:
(a₁) - einen Anteil von mehr als oder gleich 30 Mol-% und weniger als oder gleich 100 Mol-% an Monomereinheiten, die von 2-Methyl-2-[(1-oxo-2-propenyl)amino]-1-propansulfonsäure, in Form der freien Säure oder partiell oder vollständig versalzt, stammen;
(a₂) - gegebenenfalls einen Anteil von mehr als 0 Mol-% und weniger als oder gleich 70 Mol-% an Monomereinheiten, die von mindestens einem Monomer stammen, das aus den Elementen der aus Acrylsäure, Methacrylsäure, 2-Carboxyethylacrylsäure, Itaconsäure, Maleinsäure, 3-Methyl-3-[(1-oxo-2-propenyl)amino]butansäure bestehenden Gruppe, wobei die Carboxylfunktion der Monomere in Form der freien Säure, partiell versalzt oder vollständig versalzt ist, und/oder aus den Elementen der aus (2- Hydroxyethyl)acrylat, (2,3-Dihydroxypropyl)acrylat, (2-Hydroxyethyl)methacrylat, (2,3-Dihydroxypropyl)methacrylat bestehenden Gruppe oder Vinylpyrrolidon ausgewählt ist;
(a₃) - einen Anteil von mehr als 0 Mol-% und weniger als oder gleich 1 Mol-% an Monomereinheiten, die von mindestens einem diethylenisch oder polyethylenisch vernetzenden Monomer (AR) stammen;
wobei die Summe der molaren Anteile der Monomereinheiten gemäß a₁), a₂) und a₃) gleich 100 Mol-% ist;
wobei es sich bei dem inversen Latex um eine Emulsion des Wasser-in-Öl-Typs (E) handelt, die pro 100 % ihrer Masse Folgendes umfasst:
a) - von 10 Massen-% bis 90 Massen-% des vernetzten anionischen Polyelektrolyten (P);
b) - von 5 Massen-% bis 50 Massen-% einer aus mindestens einem Öl (H) bestehenden Fettphase,
c) - von 1 Massen-% bis 50 Massen-% Wasser und
d) - von 0,5 Massen-% bis 10 Massen-% eines Emulgiersystems des Wasser-in-Öl-Typs (S₁),
wobei die Summe der Massenanteile der Verbindungen gemäß a), b), c) und d) gleich 100 Massen-% ist,
wobei die Zusammensetzung (Cₑ) pro 100 % ihrer Masse Folgendes umfasst:
e₁) - von 10 Massen-% bis 60 Massen-% mindestens einer Verbindung der Formel (I):
HO-[CH₂-CH(OH)-CH₂-O]ₙ-H (I)
worin n eine ganze Zahl größer als oder gleich eins und kleiner als oder gleich fünfzehn darstellt;
e₂) - von 40 Massen-% bis 90 Massen-% mindestens einer Verbindung der Formel (II):
R₁-(C=O)-[O-CH₂-CH(OH)-CH₂]ₚ-OH (II),
worin p, das verschieden von oder gleich n ist, eine ganze Zahl größer als oder gleich eins und kleiner als oder gleich fünfzehn darstellt und die Gruppe R₁-(C=O)-einen geraden oder verzweigten, gesättigten oder ungesättigten aliphatischen Rest mit sechs bis zweiundzwanzig Kohlenstoffatomen darstellt, und gegebenenfalls
e₃) - bis zu 30 Massen-% mindestens einer Zusammensetzung (C₁₁), dargestellt durch die Formel (III):
HO-[CH₂-CHOH-CH₂-O-]_{q}-(G)ᵣ-H (III),
wobei in Formel (III) q, das verschieden von oder gleich n ist, eine ganze Zahl größer als oder gleich eins und kleiner oder gleich 3 darstellt, G den Rest eines reduzierenden Zuckers darstellt und r eine Dezimalzahl größer als oder gleich 1,05 und kleiner als oder gleich 5,00 darstellt, wobei die Zusammensetzung (C₁₁) aus einem Gemisch der Verbindungen der Formeln (III₁), (III₂), (III₃), (III₄) und (III₅) besteht:
HO-[CH₂-CHOH-CH₂-O-]_{q} -O-(G)₁-H (III₁),
HO-[CH₂-CHOH-CH₂-O-]_{q} -O-(G)₂-H (III₂),
HO-[CH₂-CHOH-CH₂-O-]_{q} -O-(G)₃-H (III₃),
HO-[CH₂-CHOH-CH₂-O-]_{q} -O-(G)₄-H (III₄),
HO-[CH₂-CHOH-CH₂-O-]_{q} -O-(G)₅-H (III₃),
in molaren Anteilen dieser Verbindungen der Formeln (III₁), (III₂), (III₃), (III₄) und (III₅) besteht, die gleich a₁, a₂, a₃, a₄ bzw. a₅ sind, so dass die Summe a₁+ a₂ + a₃ + a₄ + a₅ gleich eins ist und die Summe a₁ + 2a₂ + 3a₃ + 4a₄ + 5a₅ gleich r ist;
wobei die Summe der Massenanteile der Verbindungen gemäß e₁), e₂) und e₃) gleich 100 Massen-% ist.

10. Verwendung nach Anspruch 9, **dadurch gekennzeichnet, dass** in der Formel (I) gemäß obiger Definition n eine ganze Zahl größer als oder gleich eins und kleiner als oder gleich zehn darstellt, und dadurch, dass in der Formel (II) gemäß obiger Definition p, das gleich oder verschieden von n ist, eine ganze Zahl größer als oder gleich eins und kleiner als oder gleich zehn darstellt und die Gruppe R₁-(C=O)- aus Octanoyl-, Decanoyl-, ω-Undecylenoyl-, Dodecanoyl-, Tetradecanoyl-, Hexadecanoyl-, Octadecanoyl-, 9-Octadecenoyl- oder 9,12-Octadecadienoyl-Resten ausgewählt ist.

11. Verwendung nach einem der Ansprüche 9 oder 10, **dadurch gekennzeichnet, dass** sie pro 100 % ihrer Masse aus Folgendem besteht:
e₁) - von 10 Massen-% bis 60 Massen-% mindestens einer Verbindung der Formel (I) gemäß obiger Definition und
e₂) - von 40 Massen-% bis 90 Massen-% von mindestens einer Verbindung der Formel (II) gemäß obiger Definition.

12. Verwendung nach einem der Ansprüche 9 oder 10, **dadurch gekennzeichnet, dass** in der Formel (III) gemäß obiger Definition q gleich eins ist, G den Rest von Glucose darstellt und r eine Dezimalzahl größer als oder gleich 1,05 und kleiner als oder gleich 2,5 darstellt.

## Claims

1. Use of a self-invertible inverse latex as thickening and/or emulsifying and/or stabilizing agent for a topical cosmetic composition, said self-invertible inverse latex being a self-invertible inverse latex of a crosslinked anionic polyelectrolyte (P) comprising, per 100 mol%:
(a₁) - a proportion of greater than or equal to 30 mol% and less than or equal to 100 mol% of monomer units resulting from 2-methyl-2-[(1-oxo-2-propenyl)amino]-1-propanesulfonic acid in free acid or partially or completely salified form;
(a₂) - optionally a proportion of greater than 0 mol% and less than or equal to 70 mol% of monomer units resulting from at least one monomer chosen from the elements of the group consisting of acrylic acid, methacrylic acid, 2-(carboxyethyl)acrylic acid, itaconic acid, maleic acid and 3-methyl-3-[(1-oxo-2-propenyl)amino]butanoic acid, the carboxyl functional group of said monomers being in the free acid, partially salified or completely salified form, and/or from the elements of the group consisting of 2-hydroxyethyl acrylate, 2,3-dihydroxypropyl acrylate, 2-hydroxyethyl methacrylate, 2,3-dihydroxypropyl methacrylate, or vinylpyrrolidone;
(a₃) - a proportion of greater than 0 mol% and less than or equal to 1 mol%, of monomer units resulting from at least one diethylenic or polyethylenic crosslinking monomer (AR);
the sum of said molar proportions of monomer units according to a₁), a₂) and a₃) being equal to 100 mol%;
said self-invertible inverse latex being an emulsion of water-in-oil type (W) comprising, per 100% of its weight:
a) - from 10% by weight to 90% by weight of said crosslinked anionic polyelectrolyte (P);
b) - from 5% by weight to 50% by weight of a fatty phase constituted of at least one oil (O);
c) - from 1% by weight to 50% by weight of water;
d) - from 0.5% by weight to 10% by weight of an emulsifying system of water-in-oil type (S₁); and
e) - from 2% by weight to 10% by weight of an emulsifying system of oil-in-water type (S₂);
the sum of the proportions by weight of compounds according to a), b), c), d) and e) being equal to 100% by weight;
said self-invertible inverse latex being **characterized in that** said emulsifying system of oil-in-water type (S₂) comprises, per 100% of its weight:
f) - a proportion of greater than or equal to 50% by weight and less than or equal to 100% of a composition (Cₑ) which comprises, per 100% of its weight:
e₁) - from 10% by weight to 60% by weight of at least one compound of formula (I):
HO-[CH₂-CH(OH)-CH₂-O]ₙ-H (I)
in which n represents an integer greater than or equal to 1 and less than or equal to 15;
e₂) - from 40% by weight to 90% by weight of at least one compound of formula (II):
R₁-(C=O)-[O-CH₂-CH(OH)-CH₂]ₚ-OH (II),
in which p, which is different from or identical to n, represents an integer greater than or equal to 1 and less than or equal to 15; and in which the R₁-(C=O)- group represents a saturated or unsaturated and linear or branched aliphatic radical comprising from 6 to 22 carbon atoms; and optionally
e₃) - up to 30% by weight of at least one composition (C₁₁) represented by the formula (III):
HO-[CH₂-CHOH-CH₂-O-]_{q}-(G)ᵣ-H (III),
in which q, which is different from or identical to n, represents an integer greater than or equal to 1 and less than or equal to 3, G represents the residue of a reducing sugar and r represents a decimal number greater than or equal to 1.05 and less than or equal to 5.00;
said composition (C₁₁) consisting of a mixture of the compounds of formulae (III₁), (III₂), (III₃), (III₄) and (III₅) :
HO-[CH₂-CHOH-CH₂-O-]_{q}-O-(G)₁-H (III₁),
HO-[CH₂-CHOH-CH₂-O-]_{q}-O-(G)₂-H (III₂),
HO-[CH₂-CHOH-CH₂-O-]_{q}-O-(G)₃-H (III₃),
HO-[CH₂-CHOH-CH₂-O-]_{q}-O-(G)₄-H (III₄),
HO-[CH₂-CHOH-CH₂-O-]_{q}-O-(G)₅-H (III₅),
in molar proportions of said compounds of formulae (III₁), (III₂), (III₃), (III₄) and (III₅) respectively equal to a₁, a₂, a₃, a₄ and a₅, such that the sum (a₁ + a₂ + a₃ + a₄ + a₅) is equal to 1, and such that the sum (a₁ + 2a₂ + 3a₃ + 4a₄ + 5a₅) is equal to r;
the sum of the proportions by weight of compounds according to e₁), e₂) and e₃) being equal to 100% by weight.

2. Use as defined in Claim 1, **characterized in that** said crosslinked anionic polyelectrolyte (P) is a homopolymer of 2-methyl-2-[(1-oxo-2-propenyl)amino]-1-propanesulfonic acid partially or completely salified in the sodium salt or ammonium salt form, crosslinked by triallylamine and/or methylenebis(acrylamide); a copolymer of 2-methyl-2-[(1-oxo-2-propenyl)amino]-1-propanesulfonic acid and of acrylic acid which are partially or completely salified in the sodium salt or ammonium salt form, crosslinked by triallylamine and/or methylenebis(acrylamide); a copolymer of 2-methyl-2-[(1-oxo-2-propenyl)amino]-1-propanesulfonic acid (γ) partially or completely salified in the sodium salt form and of acrylic acid (δ) partially or completely salified in the sodium salt form in a molar ratio (γ)/(δ) of greater than or equal to 30/70 and less than or equal to 90/10, crosslinked by triallylamine and/or methylenebis(acrylamide); or a copolymer of 2-methyl-2-[(1-oxo-2-propenyl)amino]-1-propanesulfonic acid (γ) partially or completely salified in the sodium salt form and of acrylic acid (δ) partially or completely salified in the sodium salt form in a molar ratio (γ)/(δ) of greater than or equal to 40/60 and less than or equal to 90/10, crosslinked by triallylamine and/or methylenebis(acrylamide).

3. Use as defined in either one of Claims 1 and 2, **characterized in that**, in the formula (I) as defined above, n represents an integer greater than or equal to 1 and less than or equal to 10, and **in that**, in the formula (II) as defined above, p, which is identical to or different from n, represents an integer greater than or equal to 1 and less than or equal to 10, and the group R₁-(C=O)- is chosen from octanoyl, decanoyl, ω-undecylenoyl, dodecanoyl, tetradecanoyl, hexadecanoyl, octadecanoyl, 9-octadecenoyl and 9,12-octadecadienoyl radicals.

4. Use as defined in any one of Claims 1 to 3, **characterized in that**, in said emulsifying system of oil-in-water type (S₂), said composition (Cₑ) as defined above consists of, per 100% of its weight:
e₁) - from 10% by weight to 60% by weight of at least one compound of formula (I) as defined above and
e₂) - from 40% by weight to 90% by weight of at least one compound of formula (II) as defined above.

5. Use as defined in any one of Claims 1 to 3, **characterized in that**, in the formula (III) as defined above, q is equal to 1, G represents the residue of glucose and r represents a decimal number greater than or equal to 1.05 and less than or equal to 2.5.

6. Use as defined in any one of Claims 1 to 5, **characterized in that** said emulsifying system of oil-in water type (S₂) is said composition (Cₑ) as defined above.

7. Topical cosmetic composition (F), **characterized in that** it comprises, as thickening agent, per 100% of its total weight, between 0.1% and 10% by weight of a self-invertible inverse latex of a crosslinked anionic polyelectrolyte (P) comprising, per 100 mol%:
(a₁) - a proportion of greater than or equal to 30 mol% and less than or equal to 100 mol% of monomer units resulting from 2-methyl-2-[(1-oxo-2-propenyl)amino]-1-propanesulfonic acid in free acid or partially or completely salified form;
(a₂) - optionally a proportion of greater than 0 mol% and less than or equal to 70 mol% of monomer units resulting from at least one monomer chosen from the elements of the group consisting of acrylic acid, methacrylic acid, 2-(carboxyethyl)acrylic acid, itaconic acid, maleic acid and 3-methyl-3-[(1-oxo-2-propenyl)amino]butanoic acid, the carboxyl functional group of said monomers being in the free acid, partially salified or completely salified form, and/or from the elements of the group consisting of 2-hydroxyethyl acrylate, 2,3-dihydroxypropyl acrylate, 2-hydroxyethyl methacrylate, 2,3-dihydroxypropyl methacrylate, or vinylpyrrolidone;
(a₃) - a proportion of greater than 0 mol% and less than or equal to 1 mol%, of monomer units resulting from at least one diethylenic or polyethylenic crosslinking monomer (AR);
the sum of said molar proportions of monomer units according to a₁), a₂) and a₃) being equal to 100 mol%;
said self-invertible inverse latex being an emulsion of water-in-oil type (W) comprising, per 100% of its weight:
a) - from 10% by weight to 90% by weight of said crosslinked anionic polyelectrolyte (P);
b) - from 5% by weight to 50% by weight of a fatty phase constituted of at least one oil (O);
c) - from 1% by weight to 50% by weight of water;
d) - from 0.5% by weight to 10% by weight of an emulsifying system of water-in-oil type (S₁); and
e) - from 2% by weight to 10% by weight of an emulsifying system of oil-in-water type (S₂);
the sum of the proportions by weight of compounds according to a), b), c), d) and e) being equal to 100% by weight;
said self-invertible inverse latex being **characterized in that** said emulsifying system of oil-in-water type (S₂) comprises, per 100% of its weight:
f) - a proportion of greater than or equal to 50% by weight and less than or equal to 100% of a composition (Cₑ) which comprises, per 100% of its weight:
e₁) - from 10% by weight to 60% by weight of at least one compound of formula (I):
HO-[CH₂-CH(OH)-CH₂-O]ₙ-H (I)
in which n represents an integer greater than or equal to 1 and less than or equal to 15;
e₂) - from 40% by weight to 90% by weight of at least one compound of formula (II):
R₁-(C=O)-[O-CH₂-CH(OH)-CH₂]ₚ-OH (II),
in which p, which is different from or identical to n, represents an integer greater than or equal to 1 and less than or equal to 15; and in which the R₁-(C=O)- group represents a saturated or unsaturated and linear or branched aliphatic radical comprising from 6 to 22 carbon atoms; and optionally
e₃) - up to 30% by weight of at least one composition (C₁₁) represented by the formula (III):
HO-[CH₂-CHOH-CH₂-O-]_{q}-(G)ᵣ-H (III),
in which q, which is different from or identical to n, represents an integer greater than or equal to 1 and less than or equal to 3, G represents the residue of a reducing sugar and r represents a decimal number greater than or equal to 1.05 and less than or equal to 5.00;
said composition (C₁₁) consisting of a mixture of the compounds of formulae (III₁), (III₂), (III₃), (III₄) and (III₅) :
HO-[CH₂-CHOH-CH₂-O-]_{q}-O-(G)₁-H (III₁),
HO-[CH₂-CHOH-CH₂-O-]_{q}-O-(G)₂-H (III₂),
HO-[CH₂-CHOH-CH₂-O-]_{q}-O-(G)₃-H (III₃),
HO-[CH₂-CHOH-CH₂-O-]_{q}-O-(G)₄-H (III₄),
HO-[CH₂-CHOH-CH₂-O-]_{q}-O-(G)₅-H (III₅),
in molar proportions of said compounds of formulae (III₁), (III₂), (III₃), (III₄) and (III₅) respectively equal to a₁, a₂, a₃, a₄ and a₅, such that the sum (a₁ + a₂ + a₃ + a₄ + a₅) is equal to 1, and such that the sum (a₁ + 2a₂ + 3a₃ + 4a₄ + 5a₅) is equal to r;
the sum of the proportions by weight of compounds according to e₁), e₂) and e₃) being equal to 100% by weight.

8. Topical pharmaceutical composition (G), **characterized in that** it comprises, as thickening agent, per 100% of its total weight, between 0.1% and 10% by weight of a self-invertible inverse latex of a crosslinked anionic polyelectrolyte (P) comprising, per 100 mol%:
(a₁) - a proportion of greater than or equal to 30 mol% and less than or equal to 100 mol% of monomer units resulting from 2-methyl-2-[(1-oxo-2-propenyl)amino]-1-propanesulfonic acid in free acid or partially or completely salified form;
(a₂) - optionally a proportion of greater than 0 mol% and less than or equal to 70 mol% of monomer units resulting from at least one monomer chosen from the elements of the group consisting of acrylic acid, methacrylic acid, 2-(carboxyethyl)acrylic acid, itaconic acid, maleic acid and 3-methyl-3-[(1-oxo-2-propenyl)amino]butanoic acid, the carboxyl functional group of said monomers being in the free acid, partially salified or completely salified form, and/or from the elements of the group consisting of 2-hydroxyethyl acrylate, 2,3-dihydroxypropyl acrylate, 2-hydroxyethyl methacrylate, 2,3-dihydroxypropyl methacrylate, or vinylpyrrolidone;
(a₃) - a proportion of greater than 0 mol% and less than or equal to 1 mol%, of monomer units resulting from at least one diethylenic or polyethylenic crosslinking monomer (AR);
the sum of said molar proportions of monomer units according to a₁), a₂) and a₃) being equal to 100 mol%; said self-invertible inverse latex being an emulsion of water-in-oil type (W) comprising, per 100% of its weight:
a) - from 10% by weight to 90% by weight of said crosslinked anionic polyelectrolyte (P);
b) - from 5% by weight to 50% by weight of a fatty phase constituted of at least one oil (O);
c) - from 1% by weight to 50% by weight of water;
d) - from 0.5% by weight to 10% by weight of an emulsifying system of water-in-oil type (S₁); and
e) - from 2% by weight to 10% by weight of an emulsifying system of oil-in-water type (S₂);
the sum of the proportions by weight of compounds according to a), b), c), d) and e) being equal to 100% by weight;
said self-invertible inverse latex being **characterized in that** said emulsifying system of oil-in-water type (S₂) comprises, per 100% of its weight:
f) - a proportion of greater than or equal to 50% by weight and less than or equal to 100% of a composition (Cₑ) which comprises, per 100% of its weight:
e₁) - from 10% by weight to 60% by weight of at least one compound of formula (I):
HO-[CH₂-CH(OH)-CH₂-O]ₙ-H (I)
in which n represents an integer greater than or equal to 1 and less than or equal to 15;
e₂) - from 40% by weight to 90% by weight of at least one compound of formula (II):
R₁-(C=O)-[O-CH₂-CH(OH)-CH₂]ₚ-OH (II),
in which p, which is different from or identical to n, represents an integer greater than or equal to 1 and less than or equal to 15; and in which the R₁-(C=O)- group represents a saturated or unsaturated and linear or branched aliphatic radical comprising from 6 to 22 carbon atoms; and optionally
e₃) - up to 30% by weight of at least one composition (C₁₁) represented by the formula (III):
HO-[CH₂-CHOH-CH₂-O-]_{q}-(G)ᵣ-H (III),
in which q, which is different from or identical to n, represents an integer greater than or equal to 1 and less than or equal to 3, G represents the residue of a reducing sugar and r represents a decimal number greater than or equal to 1.05 and less than or equal to 5.00;
said composition (C₁₁) consisting of a mixture of the compounds of formulae (III₁), (III₂), (III₃), (III₄) and (III₅) :
HO-[CH₂-CHOH-CH₂-O-]_{q}-O-(G)₁-H (III₁),
HO-[CH₂-CHOH-CH₂-O-]_{q}-O-(G)₂-H (III₂),
HO-[CH₂-CHOH-CH₂-O-]_{q}-O-(G)₃-H (III₃),
HO-[CH₂-CHOH-CH₂-O-]_{q}-O-(G)₄-H (III₄)
HO-[CH₂-CHOH-CH₂-O-]_{q}-O-(G)₅-H (III₅),
in molar proportions of said compounds of formulae (III₁), (III₂), (III₃), (III₄) and (III₅) respectively equal to a₁, a₂, a₃, a₄ and a₅, such that the sum (a₁ + a₂ + a₃ + a₄ + a₅) is equal to 1, and such that the sum (a₁ + 2a₂ + 3a₃ + 4a₄ + 5a₅) is equal to r;
the sum of the proportions by weight of compounds according to e₁), e₂) and e₃) being equal to 100% by weight.

9. Use of a composition (Cₑ) as inverting agent of an inverse latex of a crosslinked anionic polyelectrolyte (P) comprising, per 100 mol%:
(a₁) - a proportion of greater than or equal to 30 mol% and less than or equal to 100 mol% of monomer units resulting from 2-methyl-2-[(1-oxo-2-propenyl)amino]-1-propanesulfonic acid in free acid or partially or completely salified form;
(a₂) - optionally a proportion of greater than 0 mol% and less than or equal to 70 mol% of monomer units resulting from at least one monomer chosen from the elements of the group consisting of acrylic acid, methacrylic acid, 2-(carboxyethyl)acrylic acid, itaconic acid, maleic acid and 3-methyl-3-[(1-oxo-2-propenyl)amino]butanoic acid, the carboxyl functional group of said monomers being in the free acid, partially salified or completely salified form, and/or from the elements of the group consisting of 2-hydroxyethyl acrylate, 2,3-dihydroxypropyl acrylate, 2-hydroxyethyl methacrylate, 2,3-dihydroxypropyl methacrylate, or vinylpyrrolidone;
(a₃) - a proportion of greater than 0 mol% and less than or equal to 1 mol%, of monomer units resulting from at least one diethylenic or polyethylenic crosslinking monomer (AR);
the sum of said molar proportions of monomer units according to a₁), a₂) and a₃) being equal to 100 mol%;
said inverse latex being an emulsion of water-in-oil type (W) comprising, per 100% of its weight:
a) - from 10% by weight to 90% by weight of said crosslinked anionic polyelectrolyte (P);
b) - from 5% by weight to 50% by weight of a fatty phase constituted of at least one oil (O);
c) - from 1% by weight to 50% by weight of water; and
d) - from 0.5% by weight to 10% by weight of an emulsifying system of water-in-oil type (S₁);
the sum of the proportions by weight of compounds according to a), b), c) and d) being equal to 100% by weight;
said composition (Cₑ) comprising, per 100% of its weight:
e₁) - from 10% by weight to 60% by weight of at least one compound of formula (I):
HO-[CH₂-CH(OH)-CH₂-O]ₙ-H (I)
in which n represents an integer greater than or equal to 1 and less than or equal to 15;
e₂) - from 40% by weight to 90% by weight of at least one compound of formula (II):
R₁-(C=O)-[O-CH₂-CH(OH)-CH₂]ₚ-OH (II),
in which p, which is different from or identical to n, represents an integer greater than or equal to 1 and less than or equal to 15 and the R₁-(C=O)- group represents a saturated or unsaturated and linear or branched aliphatic radical comprising from 6 to 22 carbon atoms; and optionally
e₃) - up to 30% by weight of at least one composition (C₁₁) represented by the formula (III):
HO-[CH₂-CHOH-CH₂-O-]_{q}-(G)ᵣ-H (III),
in which formula (III) q, which is different from or identical to n, represents an integer greater than or equal to 1 and less than or equal to 3, G represents the residue of a reducing sugar and r represents a decimal number greater than or equal to 1.05 and less than or equal to 5.00, said composition (C₁₁) consisting of a mixture of the compounds of formulae (III₁), (III₂), (III₃), (III₄) and (III₅):
HO-[CH₂-CHOH-CH₂-O-]_{q}-O-(G)₁-H (III₁),
HO-[CH₂-CHOH-CH₂-O-]_{q}-O-(G)₂-H (III₂),
HO-[CH₂-CHOH-CH₂-O-]_{q}-O-(G)₃-H (III₃),
HO-[CH₂-CHOH-CH₂-O-]_{q}-O-(G) ₄-H (III₄),
HO-[CH₂-CHOH-CH₂-O-]_{q}-O-(G)₅-H (III₃),
in molar proportions of said compounds of formulae (III₁), (III₂), (III₃), (III₄) and (III₅) respectively equal to a₁, a₂, a₃, a₄ and a₅, such that the sum a₁ + a₂ + a₃ + a₄ + a₅ is equal to 1, and such that the sum a₁ + 2a₂ + 3a₃ + 4a₄ + 5a₅ is equal to r;
the sum of the proportions by weight of compounds according to e₁), e₂) and e₃) being equal to 100% by weight.

10. Use as defined in Claim 9, **characterized in that**, in the formula (I) as defined above, n represents an integer greater than or equal to 1 and less than or equal to 10, and **in that**, in the formula (II) as defined above, p, which is identical to or different from n, represents an integer greater than or equal to 1 and less than or equal to 10, and the group R₁-(C=O)- is chosen from octanoyl, decanoyl, ω-undecylenoyl, dodecanoyl, tetradecanoyl, hexadecanoyl, octadecanoyl, 9-octadecenoyl and 9,12-octadecadienoyl radicals.

11. Use as defined in either one of Claims 9 and 10, **characterized in that** it consists of, per 100% of its weight:
e₁) - from 10% by weight to 60% by weight of at least one compound of formula (I) as defined above and
e₂) - from 40% by weight to 90% by weight of at least one compound of formula (II) as defined above.

12. Use as defined in either one of Claims 9 and 10, **characterized in that**, in the formula (III) as defined above, q is equal to 1, G represents the residue of glucose and r represents a decimal number greater than or equal to 1.05 and less than or equal to 2.5.
